(19) Europäisches Patentamt
European Patent Office
Office européen des brevets

(11) EP 3 004 336 B1

(12) **EUROPEAN PATENT SPECIFICATION**

(45) Date of publication and mention of the grant of the patent:
**29.03.2017 Bulletin 2017/13**

(21) Application number: **14726999.7**

(22) Date of filing: **28.05.2014**

(51) Int Cl.:
***C12N 9/10*** *(2006.01)* ***C12N 15/52*** *(2006.01)*

(86) International application number:
**PCT/EP2014/061185**

(87) International publication number:
**WO 2014/191524 (04.12.2014 Gazette 2014/49)**

(54) **ENZYMES CATALYZING THE GLYCOSYLATION OF POLYPHENOLS**

ENZYME ZUR KATALYSIERUNG DER GLYCOSYLIERUNG VON POLYPHENOLEN

ENZYMES CATALYSANT LA GLYCOSYLATION DES POLYPHÉNOLS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2013 EP 13169812**

(43) Date of publication of application:
**13.04.2016 Bulletin 2016/15**

(73) Proprietor: **Universität Hamburg**
**20148 Hamburg (DE)**

(72) Inventors:
- **RABAUSCH, Ulrich**
  **20249 Hamburg (DE)**
- **STREIT, Wolfgang**
  **24248 Mönkeberg (DE)**
- **JÜRGENSEN, Julia**
  **22851Norderstedt (DE)**

(74) Representative: **Stüven, Ralf et al**
**Pohl & Partner**
**Patentanwälte**
**Kirchenhang 32 b**
**21073 Hamburg (DE)**

(56) References cited:
**EP-A1- 2 128 265 EP-A1- 2 463 366**

- **U. RABAUSCH ET AL: "Functional Screening of Metagenome and Genome Libraries for Detection of Novel Flavonoid-Modifying Enzymes", APPLIED AND ENVIRONMENTAL MICROBIOLOGY, vol. 79, no. 15, 17 May 2013 (2013-05-17), pages 4551-4563, XP055131425, ISSN: 0099-2240, DOI: 10.1128/AEM.01077-13**
- **DATABASE EMBL [Online] 31 May 2013 (2013-05-31), "Uncultured bacterium clone fosmid pJJ144C11 glycosyltransferase (gftC) gene, complete cds.", XP002727758, retrieved from EBI accession no. EM_STD:JX157627 Database accession no. JX157627**
- **DATABASE UniProt [Online] 11 July 2012 (2012-07-11), "SubName: Full=UDP-glucuronosyl/UDP-glucosyltransfer ase;", XP002727759, retrieved from EBI accession no. UNIPROT:I2GDW4 Database accession no. I2GDW4**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 004 336 B1

SEQUENCE LISTING

<110> UniversitkÃ¤t Hamburg

<120> Enzymes catalyzing the glycosylation of polyphenols

<130> PAT 1455 PCT

<150> EP13169812.8
<151> 2013-05-29

<160> 22

<170> BiSSAP 1.3

<210> 1
<211> 1380
<212> DNA
<213> Unknown

<220>
<223> gtfC gene

<220>
<221> CDS

<222> 1..1380
<223> /trans1_table=1

<400> 1

```
atg agt aat tta ttt tct tca caa acg aac ctt gca tct gta aaa ccc       48
Met Ser Asn Leu Phe Ser Ser Gln Thr Asn Leu Ala Ser Val Lys Pro
1               5                   10                  15

ctg aaa ggc agg aaa ata ctt ttt gcc aac ttc ccg gca gat ggg cat       96
Leu Lys Gly Arg Lys Ile Leu Phe Ala Asn Phe Pro Ala Asp Gly His
            20                  25                  30

ttt aat cca ttg aca gga ctg gct gtt cac tta caa tgg ctg ggt tgt      144
Phe Asn Pro Leu Thr Gly Leu Ala Val His Leu Gln Trp Leu Gly Cys
        35                  40                  45

gat gta cgc tgg tac act tcc aat aaa tat gca gac aaa ctg cga aga      192
Asp Val Arg Trp Tyr Thr Ser Asn Lys Tyr Ala Asp Lys Leu Arg Arg
    50                  55                  60

ttg aat att ccg cat ttt cct ttc aga aaa gct atg gat ata gct gac      240
Leu Asn Ile Pro His Phe Pro Phe Arg Lys Ala Met Asp Ile Ala Asp
65                  70                  75                  80

ctg gag aat atg ttt ccg gag cgt gat gcc att aaa ggc cag gta gcc      288
Leu Glu Asn Met Phe Pro Glu Arg Asp Ala Ile Lys Gly Gln Val Ala
                85                  90                  95

aaa ctg aag ttc gac ata atc aat gct ttt att ctt cgc ggg ccg gaa      336
Lys Leu Lys Phe Asp Ile Ile Asn Ala Phe Ile Leu Arg Gly Pro Glu
            100                 105                 110

tac tat gtt gac ctg cag gag ata cat aaa agt ttt cca ttt gac gta      384
Tyr Tyr Val Asp Leu Gln Glu Ile His Lys Ser Phe Pro Phe Asp Val
        115                 120                 125

atg gtc gct gat tgc gct ttt aca gga att cct ttt gta aca gat aaa      432
Met Val Ala Asp Cys Ala Phe Thr Gly Ile Pro Phe Val Thr Asp Lys
```

```
    130                 135                 140

atg gat ata cct gtt gtt tct gta ggt gtg ttc cct ctt acc gaa aca      480
Met Asp Ile Pro Val Val Ser Val Gly Val Phe Pro Leu Thr Glu Thr
145                 150                 155                 160

tcg aaa gat ctt cct ccc gcc ggc ctc ggg att acg cct tcc ttt tct      528
Ser Lys Asp Leu Pro Pro Ala Gly Leu Gly Ile Thr Pro Ser Phe Ser
                165                 170                 175

tta ccc gga aaa ttt aaa caa agc ata cta cgg tcg gtg gct gac ctg      576
Leu Pro Gly Lys Phe Lys Gln Ser Ile Leu Arg Ser Val Ala Asp Leu
            180                 185                 190

gtc tta ttc cgc gag tcc aat aaa gta atg aga aaa atg ctg acc gaa      624
Val Leu Phe Arg Glu Ser Asn Lys Val Met Arg Lys Met Leu Thr Glu
        195                 200                 205

cat ggc att gat cat ctc tat aca aat gta ttt gac ctg atg gta aaa      672
His Gly Ile Asp His Leu Tyr Thr Asn Val Phe Asp Leu Met Val Lys
    210                 215                 220

aaa tca acg ctg cta ttg caa agc gga aca ccg ggt ttt gaa tat tac      720
Lys Ser Thr Leu Leu Leu Gln Ser Gly Thr Pro Gly Phe Glu Tyr Tyr
225                 230                 235                 240

cgc agt gat ctg gga aaa aat atc cgt ttc att ggt tca tta tta ccc      768
Arg Ser Asp Leu Gly Lys Asn Ile Arg Phe Ile Gly Ser Leu Leu Pro
                245                 250                 255

tac cag tca aaa aaa caa aca act gca tgg tct gat gaa aga ctg aac      816
Tyr Gln Ser Lys Lys Gln Thr Thr Ala Trp Ser Asp Glu Arg Leu Asn
            260                 265                 270

agg tat gaa aaa att gtg gtg gtg aca cag ggc act gtt gaa aag aat      864
Arg Tyr Glu Lys Ile Val Val Val Thr Gln Gly Thr Val Glu Lys Asn
        275                 280                 285

att gaa aag atc ctc gtg ccc act ctg gaa gcc ttt agg gat aca gac      912
Ile Glu Lys Ile Leu Val Pro Thr Leu Glu Ala Phe Arg Asp Thr Asp
    290                 295                 300

tta ttg gta ata gcc aca acg ggt gga agt ggt aca gct gag ttg aaa      960
Leu Leu Val Ile Ala Thr Thr Gly Gly Ser Gly Thr Ala Glu Leu Lys
305                 310                 315                 320

aaa aga tat cct caa ggc aac ctg atc atc gaa gat ttt att ccc ttt     1008
Lys Arg Tyr Pro Gln Gly Asn Leu Ile Ile Glu Asp Phe Ile Pro Phe
                325                 330                 335

ggc gat atc atg cct tat gcg gat gta tat att acc aat gga gga tat     1056
Gly Asp Ile Met Pro Tyr Ala Asp Val Tyr Ile Thr Asn Gly Gly Tyr
            340                 345                 350

ggt ggt gta atg ctg ggt atc gaa aac caa ttg cca ttg gta gta gcg     1104
Gly Gly Val Met Leu Gly Ile Glu Asn Gln Leu Pro Leu Val Val Ala
        355                 360                 365

ggt att cat gaa ggg aaa aat gag atc aat gca agg ata gga tac ttt     1152
Gly Ile His Glu Gly Lys Asn Glu Ile Asn Ala Arg Ile Gly Tyr Phe
    370                 375                 380

gaa ctg gga att aac ctg aaa acc gaa tgg cct aaa ccg gaa cag atg     1200
```

```
Glu Leu Gly Ile Asn Leu Lys Thr Glu Trp Pro Lys Pro Glu Gln Met
385                 390                 395                 400

aaa aaa gcc ata gat gaa gtg atc ggc aac aaa aaa tat aaa gag aat     1248
Lys Lys Ala Ile Asp Glu Val Ile Gly Asn Lys Lys Tyr Lys Glu Asn
                405                 410                 415

ata aca aaa ttg gca aaa gaa ttc agc aat tac cat ccc aat gaa cta     1296
Ile Thr Lys Leu Ala Lys Glu Phe Ser Asn Tyr His Pro Asn Glu Leu
            420                 425                 430

tgc gct cag tat ata agc gaa gta tta caa aaa aca ggc agg ctt tat     1344
Cys Ala Gln Tyr Ile Ser Glu Val Leu Gln Lys Thr Gly Arg Leu Tyr
        435                 440                 445

atc agc agt aaa aag gaa gaa gaa aag ata tac taa                     1380
Ile Ser Ser Lys Lys Glu Glu Glu Lys Ile Tyr
    450                 455
```

<210> 2
<211> 1209
<212> DNA
<213> Bacillus sp.

<220>
<223> mgtB gene

<220>
<221> CDS
<222> 1..1209
<223> /transl_table=11

<400> 2

```
atg gca aat gta ctc gta ata aat ttc cct ggg gaa ggt cat att aat       48
Met Ala Asn Val Leu Val Ile Asn Phe Pro Gly Glu Gly His Ile Asn
1               5                   10                  15

ccg act tta gct att gta agt gag tta att cag cga ggg gaa aca gtt       96
Pro Thr Leu Ala Ile Val Ser Glu Leu Ile Gln Arg Gly Glu Thr Val
            20                  25                  30

gtt tct tat tgt att gaa gat tat aga aag aag gtt gaa gca aca ggt      144
Val Ser Tyr Cys Ile Glu Asp Tyr Arg Lys Lys Val Glu Ala Thr Gly
        35                  40                  45

gcg gaa ttc cga gtg ttt gag aat ttt ctc tct caa att aat att atg      192
Ala Glu Phe Arg Val Phe Glu Asn Phe Leu Ser Gln Ile Asn Ile Met
    50                  55                  60

gaa cga gta aat gaa ggt ggg agc cct ttg atg atg cta tct cat atg      240
Glu Arg Val Asn Glu Gly Gly Ser Pro Leu Met Met Leu Ser His Met
65                  70                  75                  80

att gaa gca tca gag cgt att gtt act caa att gta gaa gaa aca aaa      288
Ile Glu Ala Ser Glu Arg Ile Val Thr Gln Ile Val Glu Glu Thr Lys
                85                  90                  95

gag gaa aaa tat gat tat tta ata tat gat aat cat ttt cca gta gga      336
Glu Glu Lys Tyr Asp Tyr Leu Ile Tyr Asp Asn His Phe Pro Val Gly
            100                 105                 110

cgt att ata gca aat att tta caa tta cca agc gtt tca tct tgt aca      384
Arg Ile Ile Ala Asn Ile Leu Gln Leu Pro Ser Val Ser Ser Cys Thr
```

```
        115                 120                 125

acg ttt gct gtt aat cag tac att aat ttt cat gat ggg caa gaa tcg      432
Thr Phe Ala Val Asn Gln Tyr Ile Asn Phe His Asp Gly Gln Glu Ser
    130                 135                 140

aga caa gta gac gaa ata aat cca tta tat caa tct tgt tta gcg gga      480
Arg Gln Val Asp Glu Ile Asn Pro Leu Tyr Gln Ser Cys Leu Ala Gly
145                 150                 155                 160

atg gaa aga tgg aat aag cac tat gga atg aaa tgt aat agt atg tat      528
Met Glu Arg Trp Asn Lys His Tyr Gly Met Lys Cys Asn Ser Met Tyr
                165                 170                 175

gat att atg aat cat cct ggt gat att acg att gta tat act tca aaa      576
Asp Ile Met Asn His Pro Gly Asp Ile Thr Ile Val Tyr Thr Ser Lys
            180                 185                 190

gaa tat cag ccg cgt tca gat tta tat gat gaa tcg tat aaa ttt gta      624
Glu Tyr Gln Pro Arg Ser Asp Leu Tyr Asp Glu Ser Tyr Lys Phe Val
        195                 200                 205

ggt cca tca att gct act cga aaa gaa gtg ggg agt ttt cct acc gaa      672
Gly Pro Ser Ile Ala Thr Arg Lys Glu Val Gly Ser Phe Pro Thr Glu
    210                 215                 220

gat tta aaa aat gaa aaa gtg att ttc att tct atg gga aca gtt ttt      720
Asp Leu Lys Asn Glu Lys Val Ile Phe Ile Ser Met Gly Thr Val Phe
225                 230                 235                 240

aat gaa caa cct gct ttg tat gaa aaa tgt ttt gaa gcg ttt aaa gat      768
Asn Glu Gln Pro Ala Leu Tyr Glu Lys Cys Phe Glu Ala Phe Lys Asp
                245                 250                 255

gta gat gcg aca gtc gta tta gtc gtt ggt aag aag ata aat aca agt      816
Val Asp Ala Thr Val Val Leu Val Val Gly Lys Lys Ile Asn Thr Ser
            260                 265                 270

caa ttt gaa aat atc ccg aaa aac ttt aag ttg tat aat tat gtc ccg      864
Gln Phe Glu Asn Ile Pro Lys Asn Phe Lys Leu Tyr Asn Tyr Val Pro
        275                 280                 285

caa tta gaa gtt tta cag cat gct gat gta ttc gtg aca cat ggt ggt      912
Gln Leu Glu Val Leu Gln His Ala Asp Val Phe Val Thr His Gly Gly
    290                 295                 300

atg aat agt tcg agt gaa gcg tta tat tac ggt gtt cca tta gtt gta      960
Met Asn Ser Ser Ser Glu Ala Leu Tyr Tyr Gly Val Pro Leu Val Val
305                 310                 315                 320

att ccg gta aca gga gat cag cca ttc gtt gca aaa cga ttg act gaa     1008
Ile Pro Val Thr Gly Asp Gln Pro Phe Val Ala Lys Arg Leu Thr Glu
                325                 330                 335

gta ggg gca ggc ata aca ctt aat cgt aac gag tta act tct gaa ttg     1056
Val Gly Ala Gly Ile Thr Leu Asn Arg Asn Glu Leu Thr Ser Glu Leu
            340                 345                 350

tta cgt gag act gta aag aaa gta atg gat gat gtg acg ttt aag gaa     1104
Leu Arg Glu Thr Val Lys Lys Val Met Asp Asp Val Thr Phe Lys Glu
        355                 360                 365

aat agt cgt aaa gtg gga gag tcg ctt aga aat gct ggt gga tat caa     1152
```

```
Asn Ser Arg Lys Val Gly Glu Ser Leu Arg Asn Ala Gly Gly Tyr Gln
    370                 375                 380

agg gca gtt gag gaa ata ttt gaa tta aaa atg aag ccg tac gta aag     1200
Arg Ala Val Glu Glu Ile Phe Glu Leu Lys Met Lys Pro Tyr Val Lys
385                 390                 395                 400

att aaa tag                                                         1209
Ile Lys
```

<210> 3
<211> 1209
<212> DNA
<213> Bacillus sp.

<220>
<223> mgtC gene

<220>
<221> CDS
<222> 1..1209
<223> /transl_table=11

<400> 3

```
atg gca aac gta ctc gta ata aat ttc cct gga gaa ggt cat ata aat      48
Met Ala Asn Val Leu Val Ile Asn Phe Pro Gly Glu Gly His Ile Asn
1               5                   10                  15

ccg act ttg gct att gta agt gag tta att cgg cga gga gag aca gtt      96
Pro Thr Leu Ala Ile Val Ser Glu Leu Ile Arg Arg Gly Glu Thr Val
            20                  25                  30

gtt tcg tat tgt att gaa gat tat aga aag aag att gaa gca aca ggt     144
Val Ser Tyr Cys Ile Glu Asp Tyr Arg Lys Lys Ile Glu Ala Thr Gly
        35                  40                  45

gca gaa ttc cga gtg ttt gag aat ttc ctc tct caa att aat att atg     192
Ala Glu Phe Arg Val Phe Glu Asn Phe Leu Ser Gln Ile Asn Ile Met
    50                  55                  60

gag cga gta aat gaa ggc ggg agt cct ttg acg atg cta tct cat atg     240
Glu Arg Val Asn Glu Gly Gly Ser Pro Leu Thr Met Leu Ser His Met
65                  70                  75                  80

att gaa gca tca gag cgt att gtt act caa att gta gaa gaa aca aaa     288
Ile Glu Ala Ser Glu Arg Ile Val Thr Gln Ile Val Glu Glu Thr Lys
                85                  90                  95

ggg gaa aag tac gat tac atg ata tac gat aat cat ttt ccg gta gga     336
Gly Glu Lys Tyr Asp Tyr Met Ile Tyr Asp Asn His Phe Pro Val Gly
            100                 105                 110

cgt att ata gcc aat gct tta aaa tta cct agc gtt tct tct tgt aca     384
Arg Ile Ile Ala Asn Ala Leu Lys Leu Pro Ser Val Ser Ser Cys Thr
        115                 120                 125

acg ttt gct ttt aat caa tat att act ttt aac gat gaa cat gaa tca     432
Thr Phe Ala Phe Asn Gln Tyr Ile Thr Phe Asn Asp Glu His Glu Ser
    130                 135                 140

aga aaa gta gat gaa acg aat cca ttg tat caa tct tgt tta gcg gga     480
Arg Lys Val Asp Glu Thr Asn Pro Leu Tyr Gln Ser Cys Leu Ala Gly
```

145 150 155 160

ata gaa aaa tgg aat aaa cag tat gga atg aaa tgt aat agt atg tat 528
Ile Glu Lys Trp Asn Lys Gln Tyr Gly Met Lys Cys Asn Ser Met Tyr
165 170 175

gat att atg aat cat cct ggt gat att act att gta tat act tca aag 576
Asp Ile Met Asn His Pro Gly Asp Ile Thr Ile Val Tyr Thr Ser Lys
180 185 190

gaa tat caa cca cgt tca gat gta ttc gat gaa tcg tat aag ttt gtc 624
Glu Tyr Gln Pro Arg Ser Asp Val Phe Asp Glu Ser Tyr Lys Phe Val
195 200 205

ggc cca tcc att gct atg cgt aaa gaa gta ggc agt ttt cct atg gaa 672
Gly Pro Ser Ile Ala Met Arg Lys Glu Val Gly Ser Phe Pro Met Glu
210 215 220

gat tta aaa gat aaa aaa ttg att ttc att tct atg gga aca gtt ttt 720
Asp Leu Lys Asp Lys Lys Leu Ile Phe Ile Ser Met Gly Thr Val Phe
225 230 235 240

aat gaa caa cct gag cta tat gaa aaa tgt ttt gaa gca ttt aaa gat 768
Asn Glu Gln Pro Glu Leu Tyr Glu Lys Cys Phe Glu Ala Phe Lys Asp
245 250 255

gca gaa gcg aca gtt ata ttg gtt gtt ggt aag aag ata aat ata agt 816
Ala Glu Ala Thr Val Ile Leu Val Val Gly Lys Lys Ile Asn Ile Ser
260 265 270

caa ttt gaa aac att ccg aat aac ttt aaa ttg ttt aat tat gtg ccg 864
Gln Phe Glu Asn Ile Pro Asn Asn Phe Lys Leu Phe Asn Tyr Val Pro
275 280 285

caa tta gaa gtg tta cag tat gct gat gta ttc gtg aca cac ggt ggc 912
Gln Leu Glu Val Leu Gln Tyr Ala Asp Val Phe Val Thr His Gly Gly
290 295 300

atg aat agt tcg agt gaa gca cta tat tac ggt gtt ccg tta gtt gta 960
Met Asn Ser Ser Ser Glu Ala Leu Tyr Tyr Gly Val Pro Leu Val Val
305 310 315 320

att ccg gta aca gga gat cag cct tta gtt gcg aaa cga gtg aat gaa 1008
Ile Pro Val Thr Gly Asp Gln Pro Leu Val Ala Lys Arg Val Asn Glu
325 330 335

gta ggg gct gga ata agg ctt aat cgt aaa gaa tta act tct gaa ttg 1056
Val Gly Ala Gly Ile Arg Leu Asn Arg Lys Glu Leu Thr Ser Glu Leu
340 345 350

tta cgt gaa gct gta gag aaa gtc gcg aat gat gta agg ttt aag gaa 1104
Leu Arg Glu Ala Val Glu Lys Val Ala Asn Asp Val Arg Phe Lys Glu
355 360 365

aat agt cgt aaa gtt gga gag tca ctt cga aat gct ggt gga tat aat 1152
Asn Ser Arg Lys Val Gly Glu Ser Leu Arg Asn Ala Gly Gly Tyr Asn
370 375 380

agg gca gtt gat gaa ata tta aaa atg aaa atg aat tca tac tca aaa 1200
Arg Ala Val Asp Glu Ile Leu Lys Met Lys Met Asn Ser Tyr Ser Lys
385 390 395 400

ctt aaa taa 1209

Leu Lys

<210> 4
<211> 1176
<212> DNA
<213> Bacillus subtilis

<220>
<223> mgtS gene

<220>
<221> CDS
<222> 1..1176
<223> /transl_table=11

<400> 4

```
atg aaa aag tac cat att tcg atg atc aat atc ccg gca tac gga cat        48
Met Lys Lys Tyr His Ile Ser Met Ile Asn Ile Pro Ala Tyr Gly His
1               5                   10                  15

gtc aat cct acg ctt gct tta gta gag aag ctt tgt gag aaa ggg cac        96
Val Asn Pro Thr Leu Ala Leu Val Glu Lys Leu Cys Glu Lys Gly His
            20                  25                  30

cgt gtc acg tac gcg acg act gag gag ttt gcg ccc gct gtt cag caa       144
Arg Val Thr Tyr Ala Thr Thr Glu Glu Phe Ala Pro Ala Val Gln Gln
        35                  40                  45

gcc ggt gga gaa gca ttg ctc tat cat aca tcc ttg aat att gat cct       192
Ala Gly Gly Glu Ala Leu Leu Tyr His Thr Ser Leu Asn Ile Asp Pro
    50                  55                  60

aag caa atc agg gag atg atg gaa aag aat gac gcg ccc ctc agc ctt       240
Lys Gln Ile Arg Glu Met Met Glu Lys Asn Asp Ala Pro Leu Ser Leu
65                  70                  75                  80

ttg aaa gaa tca ctc agc att ctg ccg cag ctt gag gag tta tat aag       288
Leu Lys Glu Ser Leu Ser Ile Leu Pro Gln Leu Glu Glu Leu Tyr Lys
                85                  90                  95

gat gat cag cct gat ctg atc atc tat gac ttt gtt gcg ctg gct ggt       336
Asp Asp Gln Pro Asp Leu Ile Ile Tyr Asp Phe Val Ala Leu Ala Gly
            100                 105                 110

aaa ttg ttt gct gaa aag ctc aat gtt ccg gtc att aag ctc tgt tcg       384
Lys Leu Phe Ala Glu Lys Leu Asn Val Pro Val Ile Lys Leu Cys Ser
        115                 120                 125

tca tat gcc caa aat gaa tcc ttt cag tta gga aat gaa gac atg ctg       432
Ser Tyr Ala Gln Asn Glu Ser Phe Gln Leu Gly Asn Glu Asp Met Leu
    130                 135                 140

aag aaa ata aaa gaa gca gag gct gaa ttt aaa gcc tac ttg gag caa       480
Lys Lys Ile Lys Glu Ala Glu Ala Glu Phe Lys Ala Tyr Leu Glu Gln
145                 150                 155                 160

gag aag ttg ccg gct gtt tca ttt gaa cag tta gct gtg ccg gaa gca       528
Glu Lys Leu Pro Ala Val Ser Phe Glu Gln Leu Ala Val Pro Glu Ala
                165                 170                 175

tta aat att gtc ttt atg ccg aag tct ttt cag att cag cat gag acg       576
Leu Asn Ile Val Phe Met Pro Lys Ser Phe Gln Ile Gln His Glu Thr
```

```
        180                 185                 190

ttc gat gac cgt ttc tgt ttt gtc ggc ccc tct ctc gga gaa cga aag      624
Phe Asp Asp Arg Phe Cys Phe Val Gly Pro Ser Leu Gly Glu Arg Lys
    195                 200                 205

gaa caa gaa ggc ctg ttg att gac aag gat gat cgc ccg ctt atg ctg      672
Glu Gln Glu Gly Leu Leu Ile Asp Lys Asp Asp Arg Pro Leu Met Leu
    210                 215                 220

att tct ttg ggt acg gcg ttt aac gca tgg ccg gaa ttt tac aag atg      720
Ile Ser Leu Gly Thr Ala Phe Asn Ala Trp Pro Glu Phe Tyr Lys Met
225                 230                 235                     240

tgc atc aag gca ttt cgg gat tct tca tgg caa gtg atc atg tcg gtt      768
Cys Ile Lys Ala Phe Arg Asp Ser Ser Trp Gln Val Ile Met Ser Val
                245                 250                 255

ggg aaa acg att gat cca gaa agc ttg gag gat att cct gct aac ttt      816
Gly Lys Thr Ile Asp Pro Glu Ser Leu Glu Asp Ile Pro Ala Asn Phe
            260                 265                 270

act att cgc caa agt gtg ccg cag ctt gag gtg tta gag aaa gct gat      864
Thr Ile Arg Gln Ser Val Pro Gln Leu Glu Val Leu Glu Lys Ala Asp
        275                 280                 285

ttg ttc atc tct cat ggc ggg atg aac agt acg atg gaa gcg atg aac      912
Leu Phe Ile Ser His Gly Gly Met Asn Ser Thr Met Glu Ala Met Asn
    290                 295                 300

gca ggt gtg ccg ctt gtc gtc att ccg caa atg tat gag caa gag ctc      960
Ala Gly Val Pro Leu Val Val Ile Pro Gln Met Tyr Glu Gln Glu Leu
305                 310                 315                     320

act gca aat cgg gtt gat gaa tta ggc ctt ggc gtt tat ttg ccg aaa     1008
Thr Ala Asn Arg Val Asp Glu Leu Gly Leu Gly Val Tyr Leu Pro Lys
                325                 330                 335

gag gaa gtg act gtt tcc agc ctg cag gaa gcg gtt cag gct gta tcc     1056
Glu Glu Val Thr Val Ser Ser Leu Gln Glu Ala Val Gln Ala Val Ser
            340                 345                 350

agt gat caa gag ctg ctc agc cgc gtc aag aat atg caa aaa gat gta     1104
Ser Asp Gln Glu Leu Leu Ser Arg Val Lys Asn Met Gln Lys Asp Val
        355                 360                 365

aaa gaa gct ggc gga gcg gag cgt gcg gca gct gag att gaa gcg ttt     1152
Lys Glu Ala Gly Gly Ala Glu Arg Ala Ala Ala Glu Ile Glu Ala Phe
    370                 375                 380

atg aaa aaa tcc gct gtc ccg cag                                     1176
Met Lys Lys Ser Ala Val Pro Gln
385                 390
```

<210> 5
<211> 1194
<212> DNA
<213> Bacillus sp.

<220>
<223> mgtT gene

<220>
<221> CDS
<222> 1..1194
<223> /transl_table=11

<400> 5

```
atg gcg cgt gtt tta ttc att aat gct gga tca gaa gga cat ata aat      48
Met Ala Arg Val Leu Phe Ile Asn Ala Gly Ser Glu Gly His Ile Asn
1               5                   10                  15

cca act tta caa gtt gta gat gaa ttg att tct cgt ggt gaa gag gtc      96
Pro Thr Leu Gln Val Val Asp Glu Leu Ile Ser Arg Gly Glu Glu Val
            20                  25                  30

gtt tat ttt tca ata gaa gct ttc agg gag cgg att gag aag aca ggt     144
Val Tyr Phe Ser Ile Glu Ala Phe Arg Glu Arg Ile Glu Lys Thr Gly
        35                  40                  45

gct act gta cga acg att gat gat caa aaa ttt ata aaa gcg ttt cta     192
Ala Thr Val Arg Thr Ile Asp Asp Gln Lys Phe Ile Lys Ala Phe Leu
    50                  55                  60

tct gga ggc aga aat tat tta cag gaa aga ata aat ggc ctt cta cat     240
Ser Gly Gly Arg Asn Tyr Leu Gln Glu Arg Ile Asn Gly Leu Leu His
65                  70                  75                  80

aca gcg gat att gta ata cct agc gtt tta gaa caa att gaa ggt gaa     288
Thr Ala Asp Ile Val Ile Pro Ser Val Leu Glu Gln Ile Glu Gly Glu
                85                  90                  95

cat ttt gat tac ata att cat gat tct atg att ggc tgt ggc cat tta     336
His Phe Asp Tyr Ile Ile His Asp Ser Met Ile Gly Cys Gly His Leu
            100                 105                 110

att gct caa atc ctt aaa ctt cca gcc ata aat tca tgc aca tct ttt     384
Ile Ala Gln Ile Leu Lys Leu Pro Ala Ile Asn Ser Cys Thr Ser Phe
        115                 120                 125

gcg cag gat gaa aaa tcc ttt gag caa atg tta ggt cat cta tca aaa     432
Ala Gln Asp Glu Lys Ser Phe Glu Gln Met Leu Gly His Leu Ser Lys
    130                 135                 140

aat atc cca gta gaa att tat gat aaa ata cag aat gat ttt caa aac     480
Asn Ile Pro Val Glu Ile Tyr Asp Lys Ile Gln Asn Asp Phe Gln Asn
145                 150                 155                 160

tta acg aag gga att gct gaa aaa tat ggt gtt gaa ata aaa tca tcg     528
Leu Thr Lys Gly Ile Ala Glu Lys Tyr Gly Val Glu Ile Lys Ser Ser
                165                 170                 175

tat gaa gtt ttc tgt aat cct gca ccc ctt act att gta tat aca att     576
Tyr Glu Val Phe Cys Asn Pro Ala Pro Leu Thr Ile Val Tyr Thr Ile
            180                 185                 190

aag gag ttc cag cct ttt ggt gat acg ttt gat gaa ata tat aaa ttt     624
Lys Glu Phe Gln Pro Phe Gly Asp Thr Phe Asp Glu Ile Tyr Lys Phe
        195                 200                 205

gta gga cca tct atc tct gca caa atg aaa aac aga gac gtt gat ttt     672
Val Gly Pro Ser Ile Ser Ala Gln Met Lys Asn Arg Asp Val Asp Phe
    210                 215                 220

act tca att gaa gaa aaa agt ccg att tat att tca tta ggt act gtt     720
Thr Ser Ile Glu Glu Lys Ser Pro Ile Tyr Ile Ser Leu Gly Thr Val
```

```
225                 230                 235                 240

ttt aat gaa gcg att gac ttt tat aaa ctg tgt atg aag gcc ttt gag      768
Phe Asn Glu Ala Ile Asp Phe Tyr Lys Leu Cys Met Lys Ala Phe Glu
                245                 250                 255

aat agt gag cat aca att gtt atg tct att ggt agt aaa aca aaa ata      816
Asn Ser Glu His Thr Ile Val Met Ser Ile Gly Ser Lys Thr Lys Ile
            260                 265                 270

agt gat cta ggc gaa att cct aaa aac ttc att gtg aaa aac tat gta      864
Ser Asp Leu Gly Glu Ile Pro Lys Asn Phe Ile Val Lys Asn Tyr Val
        275                 280                 285

ccc caa act gag ctg ctt aca tat acg aaa cta ttt att aca cac ggc      912
Pro Gln Thr Glu Leu Leu Thr Tyr Thr Lys Leu Phe Ile Thr His Gly
    290                 295                 300

ggg atg aac agt gcg cat gaa gga ctg tat aac ggg gtt ccg ctc gtt      960
Gly Met Asn Ser Ala His Glu Gly Leu Tyr Asn Gly Val Pro Leu Val
305                 310                 315                 320

gta ata ccg caa agt gca gat cag cca gta gtc gca aag caa gtg gag     1008
Val Ile Pro Gln Ser Ala Asp Gln Pro Val Val Ala Lys Gln Val Glu
                325                 330                 335

agt ctt gga gca gga ata aaa tta caa atg caa gga tta act gcg gat     1056
Ser Leu Gly Ala Gly Ile Lys Leu Gln Met Gln Gly Leu Thr Ala Asp
            340                 345                 350

caa cta agt gaa agt gta gaa atg gta tta aat aat ccg tca ttt aaa     1104
Gln Leu Ser Glu Ser Val Glu Met Val Leu Asn Asn Pro Ser Phe Lys
        355                 360                 365

gaa gtt gct ttg aat ttg aag aaa tct ttc caa aaa tca ggt gga tat     1152
Glu Val Ala Leu Asn Leu Lys Lys Ser Phe Gln Lys Ser Gly Gly Tyr
    370                 375                 380

aag gaa gct gtt gat gaa att ttt ata ttt gta ggt cag taa             1194
Lys Glu Ala Val Asp Glu Ile Phe Ile Phe Val Gly Gln
385                 390                 395
```

<210> 6
<211> 1209
<212> DNA
<213> Bacillus sp.

<220>
<223> mgtW gene

<220>
<221> CDS
<222> 1..1209
<223> /trans1_table=11

<400> 6

```
atg gca aac gta ctc gta ata aat ttc cct gga gaa ggt cat ata aat       48
Met Ala Asn Val Leu Val Ile Asn Phe Pro Gly Glu Gly His Ile Asn
1               5                   10                  15

ccg act tta gct att gta agt gag tta att cgg cga ggg gaa aca gtt       96
Pro Thr Leu Ala Ile Val Ser Glu Leu Ile Arg Arg Gly Glu Thr Val
            20                  25                  30
```

```
gtt tcg tat tgt att gaa gat tat aga aag aag att gaa gca aca ggt      144
Val Ser Tyr Cys Ile Glu Asp Tyr Arg Lys Lys Ile Glu Ala Thr Gly
        35                  40                  45

gca gaa ttc cga gtg ttt gag aat ttc ctc tct caa att aat att atg      192
Ala Glu Phe Arg Val Phe Glu Asn Phe Leu Ser Gln Ile Asn Ile Met
    50                  55                  60

gaa cga gta aat gaa ggt ggg agt cct ttg acg atg cta tct cat atg      240
Glu Arg Val Asn Glu Gly Gly Ser Pro Leu Thr Met Leu Ser His Met
65                  70                  75                  80

att gaa gca tca gag cgt att gtt act caa att gta gaa gaa aca aaa      288
Ile Glu Ala Ser Glu Arg Ile Val Thr Gln Ile Val Glu Glu Thr Lys
                85                  90                  95

ggg gaa aag tac gat tac ttg ata tac gat aat cat ttt cca gta gga      336
Gly Glu Lys Tyr Asp Tyr Leu Ile Tyr Asp Asn His Phe Pro Val Gly
            100                 105                 110

cgt att ata gcg aat gtt tta aaa tta cct agc gtt tct tct tgt aca      384
Arg Ile Ile Ala Asn Val Leu Lys Leu Pro Ser Val Ser Ser Cys Thr
        115                 120                 125

acg ttt gct ttt aat cag tac att act ttt aat gat gaa caa gaa tcg      432
Thr Phe Ala Phe Asn Gln Tyr Ile Thr Phe Asn Asp Glu Gln Glu Ser
    130                 135                 140

aga caa gta gat gaa act aat cca tta tat caa tct tgt tta gcg gga      480
Arg Gln Val Asp Glu Thr Asn Pro Leu Tyr Gln Ser Cys Leu Ala Gly
145                 150                 155                 160

atg gaa aaa tgg aat agg cag tat gga atg aaa tgt att aat atg tat      528
Met Glu Lys Trp Asn Arg Gln Tyr Gly Met Lys Cys Ile Asn Met Tyr
                165                 170                 175

gat att atg aat cat cct ggt gat att act att gta tat act tca aag      576
Asp Ile Met Asn His Pro Gly Asp Ile Thr Ile Val Tyr Thr Ser Lys
            180                 185                 190

gaa tat cag ccg cgt tca gat gta ttc gat gaa tcg tat aag ttt gtc      624
Glu Tyr Gln Pro Arg Ser Asp Val Phe Asp Glu Ser Tyr Lys Phe Val
        195                 200                 205

ggt cca tca att gct act cga aaa gaa gta gat agc ttt cct atg gaa      672
Gly Pro Ser Ile Ala Thr Arg Lys Glu Val Asp Ser Phe Pro Met Glu
    210                 215                 220

gat tta aaa gat aaa caa ttg att ttc att tct atg gga aca gtt ttt      720
Asp Leu Lys Asp Lys Gln Leu Ile Phe Ile Ser Met Gly Thr Val Phe
225                 230                 235                 240

aat gaa caa cct gag tta tat gaa aaa tgt ttt gaa gcg ttt aaa gat      768
Asn Glu Gln Pro Glu Leu Tyr Glu Lys Cys Phe Glu Ala Phe Lys Asp
                245                 250                 255

gta gaa gcg aca gtc gta tta gtt gtt ggt aag aag ata aat ata agt      816
Val Glu Ala Thr Val Val Leu Val Val Gly Lys Lys Ile Asn Ile Ser
            260                 265                 270

caa ttt gaa aac att ccg aat aac ttt aag ttg tat aat tat gtg ccg      864
Gln Phe Glu Asn Ile Pro Asn Asn Phe Lys Leu Tyr Asn Tyr Val Pro
```

```
            275                 280                 285

        caa tta gaa gta tta cag tac gct gat gta ttc gtg aca cac ggt ggt      912
        Gln Leu Glu Val Leu Gln Tyr Ala Asp Val Phe Val Thr His Gly Gly
            290                 295                 300

        atg aat agt tcg agt gaa gca cta tat tac ggt gtc ccg tta gtt gta      960
        Met Asn Ser Ser Ser Glu Ala Leu Tyr Tyr Gly Val Pro Leu Val Val
        305                 310                 315                 320

        att ccg gta aca gga gat cag cct tta gtt gcg aaa cga gtg agt gaa     1008
        Ile Pro Val Thr Gly Asp Gln Pro Leu Val Ala Lys Arg Val Ser Glu
                        325                 330                 335

        gta gga gct gga ata agg ctt aat cgt aaa gaa tta act tct gaa ttg     1056
        Val Gly Ala Gly Ile Arg Leu Asn Arg Lys Glu Leu Thr Ser Glu Leu
                    340                 345                 350

        tta cgt gag act gta aag aaa gta atg tat gat gta acg ttt aag gaa     1104
        Leu Arg Glu Thr Val Lys Lys Val Met Tyr Asp Val Thr Phe Lys Glu
                355                 360                 365

        aat agt cgc aaa gtt gga gag tca ctt cga aat gct ggt ggg tat aat     1152
        Asn Ser Arg Lys Val Gly Glu Ser Leu Arg Asn Ala Gly Gly Tyr Asn
            370                 375                 380

        aga gca gtt gat gaa ata ttt aaa atg aaa ctg aat tca tac tta aaa     1200
        Arg Ala Val Asp Glu Ile Phe Lys Met Lys Leu Asn Ser Tyr Leu Lys
        385                 390                 395                 400

        ctt aaa taa                                                         1209
        Leu Lys
```

<210> 7
<211> 459
<212> PRT
<213> Unknown

<220>
<223> [CDS]:1..1380 from SEQ ID NO 1

<400> 7

```
Met Ser Asn Leu Phe Ser Ser Gln Thr Asn Leu Ala Ser Val Lys Pro
1               5                   10                  15
Leu Lys Gly Arg Lys Ile Leu Phe Ala Asn Phe Pro Ala Asp Gly His
            20                  25                  30
Phe Asn Pro Leu Thr Gly Leu Ala Val His Leu Gln Trp Leu Gly Cys
        35                  40                  45
Asp Val Arg Trp Tyr Thr Ser Asn Lys Tyr Ala Asp Lys Leu Arg Arg
    50                  55                  60
Leu Asn Ile Pro His Phe Pro Phe Arg Lys Ala Met Asp Ile Ala Asp
65                  70                  75                  80
Leu Glu Asn Met Phe Pro Glu Arg Asp Ala Ile Lys Gly Gln Val Ala
                85                  90                  95
Lys Leu Lys Phe Asp Ile Ile Asn Ala Phe Ile Leu Arg Gly Pro Glu
            100                 105                 110
Tyr Tyr Val Asp Leu Gln Glu Ile His Lys Ser Phe Pro Phe Asp Val
        115                 120                 125
Met Val Ala Asp Cys Ala Phe Thr Gly Ile Pro Phe Val Thr Asp Lys
    130                 135                 140
Met Asp Ile Pro Val Val Ser Val Gly Val Phe Pro Leu Thr Glu Thr
145                 150                 155                 160

Ser Lys Asp Leu Pro Pro Ala Gly Leu Gly Ile Thr Pro Ser Phe Ser
                165                 170                 175
Leu Pro Gly Lys Phe Lys Gln Ser Ile Leu Arg Ser Val Ala Asp Leu
            180                 185                 190
Val Leu Phe Arg Glu Ser Asn Lys Val Met Arg Lys Met Leu Thr Glu
        195                 200                 205
His Gly Ile Asp His Leu Tyr Thr Asn Val Phe Asp Leu Met Val Lys
    210                 215                 220
Lys Ser Thr Leu Leu Leu Gln Ser Gly Thr Pro Gly Phe Glu Tyr Tyr
225                 230                 235                 240
Arg Ser Asp Leu Gly Lys Asn Ile Arg Phe Ile Gly Ser Leu Leu Pro
                245                 250                 255
Tyr Gln Ser Lys Lys Gln Thr Thr Ala Trp Ser Asp Glu Arg Leu Asn
                260                 265                 270
Arg Tyr Glu Lys Ile Val Val Val Thr Gln Gly Thr Val Glu Lys Asn
        275                 280                 285
Ile Glu Lys Ile Leu Val Pro Thr Leu Glu Ala Phe Arg Asp Thr Asp
    290                 295                 300
Leu Leu Val Ile Ala Thr Thr Gly Gly Ser Gly Thr Ala Glu Leu Lys
305                 310                 315                 320
Lys Arg Tyr Pro Gln Gly Asn Leu Ile Ile Glu Asp Phe Ile Pro Phe
                325                 330                 335
Gly Asp Ile Met Pro Tyr Ala Asp Val Tyr Ile Thr Asn Gly Gly Tyr
            340                 345                 350
Gly Gly Val Met Leu Gly Ile Glu Asn Gln Leu Pro Leu Val Val Ala
        355                 360                 365
Gly Ile His Glu Gly Lys Asn Glu Ile Asn Ala Arg Ile Gly Tyr Phe
    370                 375                 380
Glu Leu Gly Ile Asn Leu Lys Thr Glu Trp Pro Lys Pro Glu Gln Met
385                 390                 395                 400
Lys Lys Ala Ile Asp Glu Val Ile Gly Asn Lys Lys Tyr Lys Glu Asn
                405                 410                 415
Ile Thr Lys Leu Ala Lys Glu Phe Ser Asn Tyr His Pro Asn Glu Leu
            420                 425                 430
Cys Ala Gln Tyr Ile Ser Glu Val Leu Gln Lys Thr Gly Arg Leu Tyr
        435                 440                 445
Ile Ser Ser Lys Lys Glu Glu Glu Lys Ile Tyr
    450                 455
```

<210> 8

<211> 402
<212> PRT
<213> Bacillus sp.

<220>
<223> [CDS]:1..1209 from SEQ ID NO 2

<400> 8

```
Met Ala Asn Val Leu Val Ile Asn Phe Pro Gly Glu Gly His Ile Asn
1               5                   10                  15
Pro Thr Leu Ala Ile Val Ser Glu Leu Ile Gln Arg Gly Glu Thr Val
            20                  25                  30
Val Ser Tyr Cys Ile Glu Asp Tyr Arg Lys Lys Val Glu Ala Thr Gly
        35                  40                  45
Ala Glu Phe Arg Val Phe Glu Asn Phe Leu Ser Gln Ile Asn Ile Met
    50                  55                  60
Glu Arg Val Asn Glu Gly Gly Ser Pro Leu Met Met Leu Ser His Met
65                  70                  75                  80
Ile Glu Ala Ser Glu Arg Ile Val Thr Gln Ile Val Glu Glu Thr Lys
                85                  90                  95
Glu Glu Lys Tyr Asp Tyr Leu Ile Tyr Asp Asn His Phe Pro Val Gly
            100                 105                 110
Arg Ile Ile Ala Asn Ile Leu Gln Leu Pro Ser Val Ser Ser Cys Thr
```

```
        115                 120                 125
Thr Phe Ala Val Asn Gln Tyr Ile Asn Phe His Asp Gly Gln Glu Ser
    130                 135                 140
Arg Gln Val Asp Glu Ile Asn Pro Leu Tyr Gln Ser Cys Leu Ala Gly
145                 150                 155                 160
Met Glu Arg Trp Asn Lys His Tyr Gly Met Lys Cys Asn Ser Met Tyr
                165                 170                 175
Asp Ile Met Asn His Pro Gly Asp Ile Thr Ile Val Tyr Thr Ser Lys
            180                 185                 190
Glu Tyr Gln Pro Arg Ser Asp Leu Tyr Asp Glu Ser Tyr Lys Phe Val
        195                 200                 205
Gly Pro Ser Ile Ala Thr Arg Lys Glu Val Gly Ser Phe Pro Thr Glu
    210                 215                 220
Asp Leu Lys Asn Glu Lys Val Ile Phe Ile Ser Met Gly Thr Val Phe
225                 230                 235                 240
Asn Glu Gln Pro Ala Leu Tyr Glu Lys Cys Phe Glu Ala Phe Lys Asp
                245                 250                 255
Val Asp Ala Thr Val Val Leu Val Val Gly Lys Lys Ile Asn Thr Ser
            260                 265                 270
Gln Phe Glu Asn Ile Pro Lys Asn Phe Lys Leu Tyr Asn Tyr Val Pro
        275                 280                 285
Gln Leu Glu Val Leu Gln His Ala Asp Val Phe Val Thr His Gly Gly
    290                 295                 300
Met Asn Ser Ser Ser Glu Ala Leu Tyr Tyr Gly Val Pro Leu Val Val
305                 310                 315                 320
Ile Pro Val Thr Gly Asp Gln Pro Phe Val Ala Lys Arg Leu Thr Glu
                325                 330                 335
Val Gly Ala Gly Ile Thr Leu Asn Arg Asn Glu Leu Thr Ser Glu Leu
            340                 345                 350
Leu Arg Glu Thr Val Lys Lys Val Met Asp Asp Val Thr Phe Lys Glu
        355                 360                 365
Asn Ser Arg Lys Val Gly Glu Ser Leu Arg Asn Ala Gly Gly Tyr Gln
    370                 375                 380
Arg Ala Val Glu Glu Ile Phe Glu Leu Lys Met Lys Pro Tyr Val Lys
385                 390                 395                 400
Ile Lys
```

<210> 9
<211> 402
<212> PRT
<213> Bacillus sp.

<220>
<223> [CDS]:1..1209 from SEQ ID NO 3

<400> 9

```
Met Ala Asn Val Leu Val Ile Asn Phe Pro Gly Glu Gly His Ile Asn
1               5                   10                  15
Pro Thr Leu Ala Ile Val Ser Glu Leu Ile Arg Arg Gly Glu Thr Val
            20                  25                  30
Val Ser Tyr Cys Ile Glu Asp Tyr Arg Lys Lys Ile Glu Ala Thr Gly
        35                  40                  45
Ala Glu Phe Arg Val Phe Glu Asn Phe Leu Ser Gln Ile Asn Ile Met
    50                  55                  60
Glu Arg Val Asn Glu Gly Gly Ser Pro Leu Thr Met Leu Ser His Met
65                  70                  75                  80
Ile Glu Ala Ser Glu Arg Ile Val Thr Gln Ile Val Glu Glu Thr Lys
                85                  90                  95
Gly Glu Lys Tyr Asp Tyr Met Ile Tyr Asp Asn His Phe Pro Val Gly
            100                 105                 110
Arg Ile Ile Ala Asn Ala Leu Lys Leu Pro Ser Val Ser Ser Cys Thr
        115                 120                 125

Thr Phe Ala Phe Asn Gln Tyr Ile Thr Phe Asn Asp Glu His Glu Ser
    130                 135                 140
Arg Lys Val Asp Glu Thr Asn Pro Leu Tyr Gln Ser Cys Leu Ala Gly
145                 150                 155                 160
Ile Glu Lys Trp Asn Lys Gln Tyr Gly Met Lys Cys Asn Ser Met Tyr
                165                 170                 175
Asp Ile Met Asn His Pro Gly Asp Ile Thr Ile Val Tyr Thr Ser Lys
            180                 185                 190
Glu Tyr Gln Pro Arg Ser Asp Val Phe Asp Glu Ser Tyr Lys Phe Val
        195                 200                 205
Gly Pro Ser Ile Ala Met Arg Lys Glu Val Gly Ser Phe Pro Met Glu
    210                 215                 220
Asp Leu Lys Asp Lys Lys Leu Ile Phe Ile Ser Met Gly Thr Val Phe
225                 230                 235                 240
Asn Glu Gln Pro Glu Leu Tyr Glu Lys Cys Phe Glu Ala Phe Lys Asp
                245                 250                 255
Ala Glu Ala Thr Val Ile Leu Val Val Gly Lys Lys Ile Asn Ile Ser
            260                 265                 270
Gln Phe Glu Asn Ile Pro Asn Asn Phe Lys Leu Phe Asn Tyr Val Pro
        275                 280                 285
Gln Leu Glu Val Leu Gln Tyr Ala Asp Val Phe Val Thr His Gly Gly
    290                 295                 300
Met Asn Ser Ser Ser Glu Ala Leu Tyr Tyr Gly Val Pro Leu Val Val
305                 310                 315                 320
Ile Pro Val Thr Gly Asp Gln Pro Leu Val Ala Lys Arg Val Asn Glu
                325                 330                 335
Val Gly Ala Gly Ile Arg Leu Asn Arg Lys Glu Leu Thr Ser Glu Leu
            340                 345                 350
Leu Arg Glu Ala Val Glu Lys Val Ala Asn Asp Val Arg Phe Lys Glu
        355                 360                 365
Asn Ser Arg Lys Val Gly Glu Ser Leu Arg Asn Ala Gly Gly Tyr Asn
    370                 375                 380
Arg Ala Val Asp Glu Ile Leu Lys Met Lys Met Asn Ser Tyr Ser Lys
385                 390                 395                 400
Leu Lys
```

<210> 10
<211> 392
<212> PRT
<213> Bacillus subtilis

<220>
<223> [CDS]:1..1176 from SEQ ID NO 4

<400> 10

```
Met Lys Lys Tyr His Ile Ser Met Ile Asn Ile Pro Ala Tyr Gly His
1               5                   10                  15
Val Asn Pro Thr Leu Ala Leu Val Glu Lys Leu Cys Glu Lys Gly His
            20                  25                  30
Arg Val Thr Tyr Ala Thr Thr Glu Glu Phe Ala Pro Ala Val Gln Gln
        35                  40                  45
Ala Gly Gly Glu Ala Leu Leu Tyr His Thr Ser Leu Asn Ile Asp Pro
    50                  55                  60
Lys Gln Ile Arg Glu Met Met Glu Lys Asn Asp Ala Pro Leu Ser Leu
65                  70                  75                  80
Leu Lys Glu Ser Leu Ser Ile Leu Pro Gln Leu Glu Glu Leu Tyr Lys
                85                  90                  95
Asp Asp Gln Pro Asp Leu Ile Ile Tyr Asp Phe Val Ala Leu Ala Gly
            100                 105                 110
Lys Leu Phe Ala Glu Lys Leu Asn Val Pro Val Ile Lys Leu Cys Ser
        115                 120                 125
Ser Tyr Ala Gln Asn Glu Ser Phe Gln Leu Gly Asn Glu Asp Met Leu


    130                 135                 140
Lys Lys Ile Lys Glu Ala Glu Ala Glu Phe Lys Ala Tyr Leu Glu Gln
145                 150                 155                 160
Glu Lys Leu Pro Ala Val Ser Phe Glu Gln Leu Ala Val Pro Glu Ala
                165                 170                 175
Leu Asn Ile Val Phe Met Pro Lys Ser Phe Gln Ile Gln His Glu Thr
            180                 185                 190
Phe Asp Asp Arg Phe Cys Phe Val Gly Pro Ser Leu Gly Glu Arg Lys
        195                 200                 205
Glu Gln Glu Gly Leu Leu Ile Asp Lys Asp Asp Arg Pro Leu Met Leu
    210                 215                 220
Ile Ser Leu Gly Thr Ala Phe Asn Ala Trp Pro Glu Phe Tyr Lys Met
225                 230                 235                 240
Cys Ile Lys Ala Phe Arg Asp Ser Ser Trp Gln Val Ile Met Ser Val
                245                 250                 255
Gly Lys Thr Ile Asp Pro Glu Ser Leu Glu Asp Ile Pro Ala Asn Phe
            260                 265                 270
Thr Ile Arg Gln Ser Val Pro Gln Leu Glu Val Leu Glu Lys Ala Asp
        275                 280                 285
Leu Phe Ile Ser His Gly Gly Met Asn Ser Thr Met Glu Ala Met Asn
    290                 295                 300
Ala Gly Val Pro Leu Val Val Ile Pro Gln Met Tyr Glu Gln Glu Leu
305                 310                 315                 320
Thr Ala Asn Arg Val Asp Glu Leu Gly Leu Gly Val Tyr Leu Pro Lys
                325                 330                 335
Glu Glu Val Thr Val Ser Ser Leu Gln Glu Ala Val Gln Ala Val Ser
            340                 345                 350
Ser Asp Gln Glu Leu Leu Ser Arg Val Lys Asn Met Gln Lys Asp Val
        355                 360                 365
Lys Glu Ala Gly Gly Ala Glu Arg Ala Ala Ala Glu Ile Glu Ala Phe
    370                 375                 380
Met Lys Lys Ser Ala Val Pro Gln
385                 390
```

<210> 11
<211> 397
<212> PRT
<213> Bacillus sp.

<220>

<223> [CDS]:1..1194 from SEQ ID NO 5

<400> 11

```
Met Ala Arg Val Leu Phe Ile Asn Ala Gly Ser Glu Gly His Ile Asn
1               5                   10                  15
Pro Thr Leu Gln Val Val Asp Glu Leu Ile Ser Arg Gly Glu Glu Val
            20                  25                  30
Val Tyr Phe Ser Ile Glu Ala Phe Arg Glu Arg Ile Glu Lys Thr Gly
        35                  40                  45
Ala Thr Val Arg Thr Ile Asp Asp Gln Lys Phe Ile Lys Ala Phe Leu
    50                  55                  60
Ser Gly Gly Arg Asn Tyr Leu Gln Glu Arg Ile Asn Gly Leu Leu His
65                  70                  75                  80
Thr Ala Asp Ile Val Ile Pro Ser Val Leu Glu Gln Ile Glu Gly Glu
                85                  90                  95
His Phe Asp Tyr Ile Ile His Asp Ser Met Ile Gly Cys Gly His Leu
            100                 105                 110
Ile Ala Gln Ile Leu Lys Leu Pro Ala Ile Asn Ser Cys Thr Ser Phe
        115                 120                 125
Ala Gln Asp Glu Lys Ser Phe Glu Gln Met Leu Gly His Leu Ser Lys
    130                 135                 140
Asn Ile Pro Val Glu Ile Tyr Asp Lys Ile Gln Asn Asp Phe Gln Asn
145                 150                 155                 160

Leu Thr Lys Gly Ile Ala Glu Lys Tyr Gly Val Glu Ile Lys Ser Ser
                165                 170                 175
Tyr Glu Val Phe Cys Asn Pro Ala Pro Leu Thr Ile Val Tyr Thr Ile
            180                 185                 190
Lys Glu Phe Gln Pro Phe Gly Asp Thr Phe Asp Glu Ile Tyr Lys Phe
        195                 200                 205
Val Gly Pro Ser Ile Ser Ala Gln Met Lys Asn Arg Asp Val Asp Phe
    210                 215                 220
Thr Ser Ile Glu Glu Lys Ser Pro Ile Tyr Ile Ser Leu Gly Thr Val
225                 230                 235                 240
Phe Asn Glu Ala Ile Asp Phe Tyr Lys Leu Cys Met Lys Ala Phe Glu
                245                 250                 255
Asn Ser Glu His Thr Ile Val Met Ser Ile Gly Ser Lys Thr Lys Ile
            260                 265                 270
Ser Asp Leu Gly Glu Ile Pro Lys Asn Phe Ile Val Lys Asn Tyr Val
        275                 280                 285
Pro Gln Thr Glu Leu Leu Thr Tyr Thr Lys Leu Phe Ile Thr His Gly
    290                 295                 300
Gly Met Asn Ser Ala His Glu Gly Leu Tyr Asn Gly Val Pro Leu Val
305                 310                 315                 320
Val Ile Pro Gln Ser Ala Asp Gln Pro Val Val Ala Lys Gln Val Glu
                325                 330                 335
Ser Leu Gly Ala Gly Ile Lys Leu Gln Met Gln Gly Leu Thr Ala Asp
            340                 345                 350
Gln Leu Ser Glu Ser Val Glu Met Val Leu Asn Asn Pro Ser Phe Lys
        355                 360                 365
Glu Val Ala Leu Asn Leu Lys Lys Ser Phe Gln Lys Ser Gly Gly Tyr
    370                 375                 380
Lys Glu Ala Val Asp Glu Ile Phe Ile Phe Val Gly Gln
385                 390                 395
```

<210> 12
<211> 402
<212> PRT
<213> Bacillus sp.

<220>
<223> [CDS]:1..1209 from SEQ ID NO 6

<400> 12

```
Met Ala Asn Val Leu Val Ile Asn Phe Pro Gly Glu Gly His Ile Asn
1               5                   10                  15
Pro Thr Leu Ala Ile Val Ser Glu Leu Ile Arg Arg Gly Glu Thr Val
            20                  25                  30
Val Ser Tyr Cys Ile Glu Asp Tyr Arg Lys Lys Ile Glu Ala Thr Gly
        35                  40                  45
Ala Glu Phe Arg Val Phe Glu Asn Phe Leu Ser Gln Ile Asn Ile Met
    50                  55                  60
Glu Arg Val Asn Glu Gly Gly Ser Pro Leu Thr Met Leu Ser His Met
65                  70                  75                  80
Ile Glu Ala Ser Glu Arg Ile Val Thr Gln Ile Val Glu Glu Thr Lys
                85                  90                  95
Gly Glu Lys Tyr Asp Tyr Leu Ile Tyr Asp Asn His Phe Pro Val Gly
            100                 105                 110
Arg Ile Ile Ala Asn Val Leu Lys Leu Pro Ser Val Ser Ser Cys Thr
        115                 120                 125
Thr Phe Ala Phe Asn Gln Tyr Ile Thr Phe Asn Asp Glu Gln Glu Ser
    130                 135                 140
Arg Gln Val Asp Glu Thr Asn Pro Leu Tyr Gln Ser Cys Leu Ala Gly
145                 150                 155                 160
Met Glu Lys Trp Asn Arg Gln Tyr Gly Met Lys Cys Ile Asn Met Tyr
                165                 170                 175
Asp Ile Met Asn His Pro Gly Asp Ile Thr Ile Val Tyr Thr Ser Lys


            180                 185                 190
Glu Tyr Gln Pro Arg Ser Asp Val Phe Asp Glu Ser Tyr Lys Phe Val
        195                 200                 205
Gly Pro Ser Ile Ala Thr Arg Lys Glu Val Asp Ser Phe Pro Met Glu
    210                 215                 220
Asp Leu Lys Asp Lys Gln Leu Ile Phe Ile Ser Met Gly Thr Val Phe
225                 230                 235                 240
Asn Glu Gln Pro Glu Leu Tyr Glu Lys Cys Phe Glu Ala Phe Lys Asp
                245                 250                 255
Val Glu Ala Thr Val Val Leu Val Val Gly Lys Lys Ile Asn Ile Ser
            260                 265                 270
Gln Phe Glu Asn Ile Pro Asn Asn Phe Lys Leu Tyr Asn Tyr Val Pro
        275                 280                 285
Gln Leu Glu Val Leu Gln Tyr Ala Asp Val Phe Val Thr His Gly Gly
    290                 295                 300
Met Asn Ser Ser Ser Glu Ala Leu Tyr Tyr Gly Val Pro Leu Val Val
305                 310                 315                 320
Ile Pro Val Thr Gly Asp Gln Pro Leu Val Ala Lys Arg Val Ser Glu
                325                 330                 335
Val Gly Ala Gly Ile Arg Leu Asn Arg Lys Glu Leu Thr Ser Glu Leu
            340                 345                 350
Leu Arg Glu Thr Val Lys Lys Val Met Tyr Asp Val Thr Phe Lys Glu
        355                 360                 365
Asn Ser Arg Lys Val Gly Glu Ser Leu Arg Asn Ala Gly Gly Tyr Asn
    370                 375                 380
Arg Ala Val Asp Glu Ile Phe Lys Met Lys Leu Asn Ser Tyr Leu Lys
385                 390                 395                 400
Leu Lys
```

<210> 13
<211> 20

<212> DNA
<213> Artificial Sequence

<220>
<223> Primer cfn_GT-1for

<400> 13
ttatgtcccg caattagaag 20

<210> 14
<211> 19
<212> DNA
<213> Artificial Sequence

<220>
<223> Primer cfn_GT-for

<400> 14
agaaggttga agcaacagg 19

<210> 15
<211> 29
<212> DNA
<213> Artificial Sequence

<220>
<223> Primer cfn_GT-rev

<400> 15
cctactggaa aatgattatc atatattac 29

<210> 16
<211> 29
<212> DNA
<213> Artificial Sequence

<220>
<223> Primer gtf-Nde-for

<400> 16
catatgagta atttattttc ttcacaaac 29

<210> 17
<211> 27
<212> DNA
<213> Artificial Sequence

<220>
<223> Primer gtf-Bam-rev

<400> 17
ggatccttag tatatctttt cttcttc 27

<210> 18
<211> 22
<212> DNA
<213> Artificial Sequence

<220>
<223> Primer mgt-1-XhoI-for

<400> 18
ctcgagatgg caaatgtact cg 22

<210> 19
<211> 25
<212> DNA
<213> Artificial Sequence

<220>
<223> Primer mgt-1-XhoI-rev

<400> 19
ctcgagttta atctttacgt acggc 25

<210> 20
<211> 17
<212> DNA
<213> Artificial Sequence

<220>
<223> T3 promoter

<400> 20
attaaccctc actaaag 17

<210> 21
<211> 19
<212> DNA
<213> Artificial Sequence

<220>
<223> T7 promoter

<400> 21
taatacgact cactatagg 19

<210> 22
<211> 19
<212> DNA
<213> Artificial Sequence

<220>
<223> T7 terminator

<400> 22
gctagttatt gctcagcgg 19

## Description

The invention relates to enzymes catalyzing the glycosylation of polyphenols, in particular flavonoids, benzoic acid derivatives, stilbenoids, chalconoids, chromones, and coumarin derivatives.

Polyphenols are secondary plant metabolites biosythesized via the Shikimic acid and phenylpropanoid pathway. They are aromatic compounds having hydroxyl groups at their ring system, or derivatives thereof. Flavonoids and benzoic acid derivatives are examples of polyphenols. Via secondary modification of the hydroxyl group(s) of the ring system a wide variety of natural derivatives of these compounds is formed. Sugar modifications frequently occur in nature, because they can have a significant impact on the solubility and the function of the compounds. Polyphenolic compounds are part of our daily nutrition in form of fruits and vegetables, and are known to have a positive influence on human health. Besides antioxidative and radical scavenging function they can act e.g. antiallergenic, antibacterial, antifungal, antiviral, antiinflammatory, analgesic, and even cancer protective (21). Because of these broad effects there is an increasing demand for polyphenols, e.g. specific flavonoids, in the cosmetic, the pharma- and nutraceutical industries (22-24). Meeting this demand a major problem arises from their limited availability. Flavonoids, for example, are exclusively produced in plants at low levels. The extraction is linked to the use of large quantities of solvents, and the chemical modification is not easily accomplished due to their rather complex structure (25).

The regio-specific modification of polyphenols such as flavonoids remains difficult as the directed chemical modification mostly fails. Thus enzymes have gained interest as they are able to mediate the regio- and stereochemical modification of polyphenols (26). In particular, research focusses on the specific glycosylation as a modification to influence water solubility and bioavailability of polyphenos such as, for example, flavonoids (27, 28). Enzymes that catalyze this reaction are glycosyltransferases (GTs). Generally, GTs mediate the transfer of sugar residues from a donor substrate to acceptor molecules. Based on their sequence similarities GTs are currently classified into 94 families (29). The GT family 1 (GT1) comprises enzymes that catalyze the glycosylation of small lipophilic molecules (30). These enzymes (EC 2.4.1.x) that use a nucleotide-activated donor belong to the UDP-glycosyltransferase (UGT) superfamily and are also referred as Leloir enzymes (31, 32). Glycosyltransferases acting on flavonoids also belong to GT1 (33). Enzymes of GT1 possess a GT-B fold structure and present an inverting reaction mechanism concerning the linkage of the transferred sugar moiety (34). EP 2 128 265 A1 describes glycosyltransferases of fungal origin, namely from the genus *Trichoderma*, for the glycosylation of flavonoids. EP 1 985 704 A1 discloses glycosyltransferases from rose plants, also acting on flavonoids. Up to now very few flavonoid-acting GT1s of prokaryotic origin have been identified and characterized in detail. The currently known flavonoid accepting UGTs derived from Gram-positive bacteria all belong to the macroside glycosyltransferase (MGT) subfamily and originate from Bacilli and Streptomycetes (35-37; see also EP 1 867 729 A1 and WO 2009/015268 A1). Furthermore a single flavonoid acting UGT derived from the Gram-negative *Xanthomonas campestris* is known (38).

Consequently, there is still a need for means for modifying polypenols like flavonoids, chromones and the like. It is therefore an object of the invention to provide such means. The object is solved by the subject-matter of the independent claims. Advantageous embodiments of the invention are specified in the dependent claims.

In a first aspect the invention provides an enzyme catalyzing the glycosylation of polyphenols such as, for example, phenolic acid derivatives, chalconoids, chromones, coumarin derivatives, flavonoids, and stilbenoids, wherein the enzyme

a) comprises the amino acid sequence according to SEQ ID NO: 7, or
b) is encoded by a nucleic acid comprising the nucleotide sequence of SEQ ID NO: 1, or
c) is at least 75 % homologous to one of the enzymes defined in a) or b) above, or
d) is encoded by a nucleic acid hybridizing under stringent conditions with a nucleic acid complementary to a sequence comprising the nucleotide sequence of SEQ ID NO: 1.

The novel enzyme described herein, designated GtfC, belongs to GT family 1 and is highly active on polyphenols like flavonoids and similar molecules.

The term "comprising" as used herein encompasses the term "having", i.e. is not to be construed as meaning that further elements have necessarily to be present in an embodiment in addition to the element explicitly mentioned. For example, the term "enzyme comprising an amino acid sequence according to SEQ ID NO:X" also encompasses an enzyme having the amino acid sequence according to SEQ ID NO:X, "having" in this context meaning being exclusively composed of the amino acids in SEQ ID NO:X.

The term "homologous" as used herein in reference to a nucleic acid, protein or peptide means that a nucleic acid is in its nucleotide sequence essentially identical or similar to another nucleic acid, or a protein or peptide is in its amino acid sequence essentially identical or similar to another protein or peptide, without being completely identical to the nucleic acid or protein or peptide with which it is compared. The presence of homology between two nucleic acids or proteins or peptides can be determined by comparing a position in the first sequence with a corresponding position

in the second sequence in order to determine whether identical or similar residues are present at that position. Two compared sequences are homologous to each other when a certain minimum percentage of identical or similar nucleotides or amino acids are present. Identity means that when comparing two sequences at equivalent positions the same nucleotide or amino acid is present. It may optionally be necessary to take sequence gaps into account in order to produce the best possible alignment. Similar amino acids are non-identical amino acids with the same or equivalent chemical and/or physical properties. The replacement of an amino acid with another amino acid with the same or equivalent physical and/or chemical properties is called a "conservative substitution". Examples of physicochemical properties of an amino acid are hydrophobicity or charge. In connection with nucleic acids it is referred to a similar nucleotide or a conservative substitution when, in a coding sequence, a nucleotide within a codon is replaced with another nucleotide, the new codon, e.g. due to the degeneracy of the genetic code, still encoding the same or a similar amino acid. The skilled person knows which nucleotide or amino acid substitution is a conservative substitution. To determine the degree of similarity or identity between two nucleic acids it is preferable to take a minimum length of 60 nucleotides or base pairs, preferably a minimum length of 70, 80, 90, 100, 110, 120, 140, 160, 180, 200, 250 , 300, 350 or 400 nucleotides or base pairs, or a length of at least 25%, 30%, 40%, 50%, 60%, 70%, 80%, 90%, 91%, 92%, 93%, 94%, 95%, 96%, 97%, 98%, 99%, 99.2% or 99.5% of the nucleotides in the respective nucleotide sequences. For proteins/peptides it is preferable to take a minimum length of 20, preferably a minimum length of 25, 30, 35, 40, 45, 50, 60, 80 or 100, more preferably a minimum length of 120, 140, 160, 180 or 200 amino acids, or a minimum length of 25%, 30%, 40%, 50%, 60%, 70%, 80%, 90%, 91%, 92%, 93%, 94%, 95%, 96%, 97%, 98%, 99%, 99.2% or 99.5% of the amino acids of the respective amino acid sequences compared. Particularly preferably the full length of the respective protein(s) or nucleic acid(s) is used for comparison. The degree of similarity or identity of two sequences can, for example, be determined by using the computer program BLAST (19), see, e.g. http://www.ncbi.nlm.nih.govBLAST/) using standard parameters. A determination of homology is dependent on the length of the sequences being compared. For the purposes of the present invention two nucleic acids, the shorter of which comprises at least 100 nucleotides, will be considered homologous when at least 70%, at least 75%, at least 80%, at least 85%, at least 90%, at least 95%, at least 96% , at least 97%, at least 98%, at least 99%, at least 99.2% or 99.5% of the nucleotides are identical and/or similar ("identities" or "positives" according to BLAST), preferably identical. In case of a sequence length of 50-99 nucleotides two nucleic acids are considered homologous when at least 80%, preferably at least 85%, 86%, 87%, 88%, 89%, or 90% of the nucleotides are identical and/or similar. In case of a sequence length of 15-49 nucleotides two nucleic acids are considered homologous when at least 90%, preferably at least 95%, 96%, 97%, 98%, 99%, 99.2% or 99.5% of the nucleotides are identical and/or similar. In the case of nucleic acids coding for a protein or peptide homology is assumed to exist if the translated amino acid sequences are homologous. As similar amino acids especially those non-identical amino acids are considered, which, on the basis of the computer program "Basic Local Alignment Search Tool", abbreviated as BLAST (19); see e.g. http://www.ncbi.nlm.nih.gov/BLAST/) using the BLOSUM62 substitution matrix (Henikoff, S. and Henikoff, J. Amino acid substitution matrices from protein blocks. Proc Natl. Acad. Sci. USA 89: 10915-10919, 1992) are designated as "positive", i.e. have a positive score in the BLOSUM62 substitution matrix. For the purposes of the present invention, it is assumed that a homology between two amino acid sequences is present if at least 55%, preferably at least 60%, at least 65%, at least 70%, at least 75%, at least 80%, at least 85%, at least 90%, at least 95%, at least 97%, at least 98%, at least 99%, at least 99.2% or at least 99.5% of the amino acids are identical or similar, preferably identical. In particular, a homology between two sequences is assumed to exist, when, using the computer program BLAST (19); see, e.g. http://www.ncbi.nlm.nih.gov/BLAST/) using standard parameters and the BLOSUM62 substitution matrix (20) an identity or similarity ("positives"), preferably identity, of at least 55%, preferably at least 60%, at least 65%, at least 70%, at least 75%, at least 80%, at least 85%, at least 90%, at least 95%, at least 97%, at least 98%, at least 99%, at least 99.2% or at least 99.5% is obtained. The skilled person, using his expert knowledge, will readily determine which of the available BLAST programs, eg BLASTp or PLASTn, is suitable for determination of homology. In addition, the skilled person is aware of further programs for assessing homology, which he may use if necessary. Such programs are, for example, available on the website of the European Bioinformatics Institute (EMBL) (see, e.g http://www.ebi.ac.uk/Tools/similarity.html). Where such terms like "x % homologous to" or "homology of x %" are used herein, this is to be construed as meaning that two proteins or nucleic acids are considered homologous and have a sequence similarity or identity, preferably identity, of x %, e.g. 80%.

The term "hybridization" is used herein in reference to the pairing of complementary nucleic acids. Hybridization and the strength of hybridization (i.e., the strength of the association between the nucleic acids) is influenced by such factors as the degree of complementary between the nucleic acids, stringency of the conditions involved, the Tm ("melting temperature") of a nucleic acid of the formed hybrid, and the G:C ratio within the nucleic acids.

The term "hybridizing under stringent conditions" refers to conditions of high stringency, i.e. in term of temperature, ionic strength, and the presence of other compounds such as organic solvents, under which nucleic acid hybridizations are conducted. With "high stringency" conditions, nucleic acid base pairing will occur only between nucleic acids having a high frequency of complementary base sequences. Stringent hybridization conditions are known to the skilled person (see e.g. Green M.R., Sambrook, J., Molecular Cloning: A Laboratory Manual, Cold Spring Harbor Laboratory

Press; 4th edition, 2012). An example for stringent hybridization conditions is hybridizing at 42° C in a solution consisting of 5× SSPE (43.8 g/l NaCl, 6.9 g/l $NaH_2PO_4H_2O$ and 1.85 g/l EDTA, pH adjusted to 7.4 with NaOH), 0.5% SDS, 5× Denhardt's reagent and 100 μg/ml denatured salmon sperm DNA followed by washing in a solution comprising 0.1×SSPE, 1.0% SDS at 42° C when a probe of about 500 nucleotides in length is employed.

The term "glycosylation" relates to a reaction in which a carbohydrate as a glycosyl donor is attached to a hydroxyl or other functional group of another molecule (a glycosyl acceptor).

The term "glycosyl donor" relates to a carbohydrate, e.g. a mono- or oligosaccharide, reacting with a suitable acceptor compound to form a new glycosidic bond.

The term "carbohydrate" comprises hydrates of carbon, i.e. a compound having the stoichiometric formula $C_n(H_2O)_n$. The generic term includes monosaccharides, oligosaccharides and polysaccharides as well as substances derived from monosaccharides by reduction of the carbonyl group (alditols), by oxidation of one or more terminal groups to carboxylic acids, or by replacement of one or more hydroxy group(s) by a hydrogen atom, an amino group, thiol group or similar groups. It also includes derivatives of these compounds. See IUPAC. Compendium of Chemical Terminology, 2nd ed. (the "Gold Book"). Compiled by A. D. McNaught and A. Wilkinson. Blackwell Scientific Publications, Oxford (1997). XML on-line corrected version: http://goldbook.iupac.org (2006-) created by M. Nic, J. Jirat, B. Kosata; updates compiled by A. Jenkins. ISBN 0-9678550-9-8. doi:10.1351/goldbook. Last update: 2014-02-24; version: 2.3.3; doi:10.1351/goldbook.C00820.

The term "polyphenols" relates to secondary plant metabolites which are biosynthesized via the Shikimic acid and phenylpropanoid pathway, and which are aromatic compounds having one, two or more hydroxyl groups directly bound to their ring system, or derivatives thereof. Examples for polyphenols are flavonoids, benzoic acid derivatives, stilbenoids, chalcones, chromones, and coumarin derivatives.

The term "flavonoid" relates to a group of compounds comprising flavones, derived from 2-phenylchromen-4-one (2-phenyl-1,4-benzopyrone) (e.g. quercetin, rutin), isoflavonoids, derived from 3-phenylchromen-4-one (3-phenyl-1,4-benzopyrone), and neoflavonoids, derived from 4-phenylcoumarine (4-phenyl-1,2-benzopyrone). The term comprises e.g flavones (e.g. luteolin, apigenin), flavanones (e.g. hesperetin, naringenin, eriodictyol), flavonols (e.g. morin, quercetin, rutin, kaempferol, myricetin, isorhamnetin, fisetin), flavanols (e.g. catechin, gallocatechin, epicatechin, epigallocatechingallat), flavanonols (e.g. taxifolin), chalcones (chalcone derivatives, e.g. isoliquiritigenin, phloretin, xanthohumol), isoflavones (e.g. genistein, daidzein, licoricidin), chromones, i.e. derivatives of chromone (1,4-benzopyrone, chromen-4-one), in particular hydroxylated chromone derivatives (e.g. noreugenin), anthocyanidins (e.g. cyanidin, delphinidin, malvidin, pelargonidin, peonidin, petunidin), and aurones (e.g. aureusidin), and acylated, glycosylated, methoxylated, and sulfoylated derivatives of the afore-mentioned compound classes. See also: IUPAC. Compendium of Chemical Terminology, 2nd ed. (the "Gold Book"). Compiled by A. D. McNaught and A. Wilkinson. Blackwell Scientific Publications, Oxford (1997). XML on-line corrected version: http://goldbook.iupac.org (2006-) created by M. Nic, J. Jirat, B. Kosata; updates compiled by A. Jenkins. ISBN 0-9678550-9-8. doi:10.1351/goldbook. Last update: 2014-02-24; version: 2.3.3; doi:10.1351/goldbook.F02424.

The term "stilbenoids" relates to hydroxylated derivatives of stilbene, and derivatives thereof, an examples being resveratrol.

The term "coumarins" relates to derivatives, in particular hydroxylated derivatives of coumarin (2H-chromen-2-one, 1-benzopyran-2-one), e.g. 7-hydroxy-4-methylcoumarin (4-MU, 4-methylumbelliferone). See also: IUPAC. Compendium of Chemical Terminology, 2nd ed. (the "Gold Book"). Compiled by A. D. McNaught and A. Wilkinson. Blackwell Scientific Publications, Oxford (1997). XML on-line corrected version: http://goldbook.iupac.org (2006-) created by M. Nic, J. Jirat, B. Kosata; updates compiled by A. Jenkins. ISBN 0-9678550-9-8. doi:10.1351/goldbook. Last update: 2014-02-24; version: 2.3.3; doi:10.1351/goldbook.C01369.

The term "benzoic acid derivatives" relates to derivatives, in particular hydroxylated derivatives of benzoic acid.

An enzyme being homologous to the enzyme of the invention is at least 75%, more preferably at least 80% or 85%, most preferred at least 90%, 95%, 96%, 97%, 98%, 99%, 99.2% or at least 99.5% homologous to the enzyme defined in a) or b) above.

In a second aspect the invention also relates to fragments of an enzyme being at least 97 % homologous to the enzyme according to the first aspect, wherein the fragment comprises at least 60, preferably at least 65, 70, 75, 80, 85, 90, 100, 110 or at least 120 consecutive amino acids of said enzyme, and wherein the fragment catalyzes the glycosylation of a polyphenol.

In a third aspect the invention relates to a nucleic acid encoding an enzyme according to the first aspect of the invention or a fragment according to the second aspect of the invention. Preferably, the nucleic acid

a) comprises the nucleotide sequence according to SEQ ID NO: 1, or a fragment thereof, the fragment encoding a fragment of an enzyme catalyzing the glycosylation of a polyphenol, or
b) is homologous to one of the nuleic acids defined in a) above, or
c) hybridizes under stringent conditions with a nucleic acid complementary to the nuleic acid defined in a) above.

In case the invention relates to a fragment of a nucleic acid or a fragment of an enzyme it is understood that the fragment, in case of nucleic acid, encodes a peptide catalyzing the glycosylation of a polyphenol or, in case of a peptide, catalyses the glycosylation of a polyphenol.

The nucleic acid, or a fragment thereof, may be incorporated in a vector such as a plasmid as a means to introduce the nucleic acid into a host cell, e.g. a fungal, bacterial or plant cell. The nucleid acid may be functionally linked to a suitable promoter and/or other regulatory sequence(s) in order to achieve expression of the nucleic acid, or fragment, in the cell. A broad variety of suitable vectors and methods for introducing such vectors into a host cell is known to the skilled person.

In a fourth aspect the invention relates to the use of an enzyme according to the first aspect or the use of a fragment according to the second aspect of the invention for the glycosylation of polyphenols, preferably phenolic acid derivatives, flavonoids, benzoic acid derivatives, stilbenoids, chalconoids, chromones, and coumarin derivatives.

In a fifth aspect the invention relates to a method for preparing a glycoside of a polyphenol, preferably a flavonoid, benzoic acid derivative, stilbenoid, chalconoid, chromone, or coumarin derivative, comprising the step of reacting the polyphenol and a glycosyl donor with an enzyme according to the first aspect of the invention or a fragment thereof according to the second aspect of the invention, under suitable conditions for an enzymatic reaction to occur transferring the glycosyl donor to a hydroxyl group or other functional group of the polyphenol.

Suitable conditions for an enzymatic glycosylation reaction are well known to the skilled person, and can also be derived from the following examples and prior art documents referenced herein.

The invention is now described for illustrative purposes only by means of the following examples.

Bacterial strains, plasmids and chemical reagents

Bacterial strains and plasmids used in the present work are listed in TABLE S1 and primers are listed in TABLE S2 below.

TABLE S1 Bacterial strains, vectors and constructs used

| **Designation** | **Genotype** | **Reference/Source** |
|---|---|---|
| *Bacillus* sp. HH1500 | Bacillus cereus group soil isolate, wild type | |
| *E. coli* BL21 (DE3) | F- ompT dcm lon hsdS(rB- mB-) ΔgalM- | (73), Merck KGaA, |
| | ybhJ λ(DE3) | Darmstadt, Germany |
| *E. coli* DH5α | F- φ80 lacZΔM15 Δ(lacZYA-argF)U169 recA1 endA1 hsdR17(rk-, mk+) phoA supE44 λ- thi-1 gyrA96 relA1 | Life Technologies, Frankfurt, Germany |
| *E. coli* EPI300TM-T1$^R$ | F- mcrAD (mrr-hsdRMS-mcrBC) Φ80dlacZΔM15 ΔlacX74 recAl endAl araD139 Δ(ara, leu)7697 galU galK λ- rpsL nupG trfA tonA dhfr | Epicentre, Madison, WI, USA |
| *p*Bluescript II SK (+) | 3.0 kb phagemid vector, lacZ, bla, PT7, PT3 | Stratagene, LaJolla, CA, USA |
| *p*CC1FosTM | 8.1 kb fosmid cloning vector, CmR, lacZ, PT7, repE, redF, parA, parB, parC, loxP | Epicentre, Madison, WI, USA |
| *p*Drive | 3.85 kb TA-cloning vector, lacZ, bla, KanR, PT7, PSP6 | Qiagen, Hilden, Germany |
| *p*D*gtfC* | 5.2 kb construct of *p*Drive and *gtfC* derived by PCR from *p*FOS144C11 using primer pair gtf-Nde-for and gtf-Bam-rev (TABLE S2) | |
| *p*D*mgtB* | 5.1 kb construct of *p*Drive and *mgtB* derived by PCR from pFOS4B2 using primer pair mgt-1-*Xho*I-for and mgt-1-*Xho*I-rev (TABLE S2) | |
| *p*ET19b | 5.7 kb, overexpression vector, PT7, lacI, bla | Merck KGaA, Darmstadt, Germany |
| *p*ET19*gtfC* | 7.1 kb construct of pET19b::*gtfC* using NdeI and BamHI sites | |
| *p*ET19*mgtB* | 6.9 kb construct of pET19b::*mgtB* using XhoI site | |
| *p*FOS4B2 | 46 kb fosmid from the *B*.sp.HH1500 library conferring glycosyltransferase activity | |
| *p*FOS19G2 | 45 kb fosmid derived from the B.sp.HH1500 library conferring glycosyltransferase activity | |

(continued)

| Designation | Genotype | Reference/Source |
|---|---|---|
| *p*FOS144C11 | 40 kb fosmid from the Elbe river sediment metagenome library conferring glycosyltransferase activity | |
| pSK4B2 | 6.2 kb HindIII-subclone ofpFOS4B2 in pBluescript II SK(+) | |
| pSK144C11 | 11.5 kb HindIII-subclone of pFOS144C11 in pBluescript II SK (+) | |
| pTZ19R-Cm | 3.1 kb, pTZ19R Δbla(CmR), PT7, lacZ (74) | |
| pTZ144E1 | 4.0 kb EcoRI-subclone of pSK144C11 in pTZ19R-Cm | |
| pTZ144E3 | 4.6 kb EcoRI-subclone of pSK144C11 in pTZ19R-Cm | |
| pTZ144P1 | 5.7 kb PstI-subclone of pSK144C11 in pTZ19R-Cm | |
| pTZ144P2 | 3.9 kb PstI-subclone of PSK144C11 in pTZ19R-Cm | |

TABLE S2 Oligonucleotides and primers used for gene amplification and sequence analysis. Recognition sites of restriction endonucleases are underlined (ID = SEQ ID NO:.

| ID: | Primer | Sequence (5'-3') | Tm [° C] | GC (%) |
|---|---|---|---|---|
| 13 | cfn_GT-1 for | TTATGTCCCGCAATTAGAAG | 53.2 | 40 |
| 14 | cfn_GT-for | AGAAGGTTGAAGCAACAGG | 54.5 | 47.4 |
| 15 | cfn_GT-rev | CCTACTGGAAAATGATTATCATATATTAC | 58.2 | 27.6 |
| 16 | gtf-*Nde*-for | CATATGAGTAATTTATTTTCTTCACAAAC | 56.8 | 24.1 |
| 17 | gtf-*Bam*-rev | GGATCCTTAGTATATCTTTTCTTCTTC | 58.9 | 33.3 |
| 18 | mgt-1-XhoI-for | CTCGAGATGGCAAATGTACTCG | 60.4 | 50 |
| 19 | mgt-1-*Xho*I-rev | CTCGAGTTTAATCTTTACGTACGGC | 61.3 | 44 |
| 20 | T3 promoter | ATTAACCCTCACTAAAG | 50.0 | 42.1 |
| 21 | T7 promoter | TAATACGACTCACTATAGG | 53.3 | 36.8 |
| 22 | T7 terminator | GCTAGTTATTGCTCAGCGG | 60,2 | 52.6 |

If not otherwise stated *Escherichia coli* was grown at 37°C in LB medium (1% tryptone, 0.5% yeast extract, 0.5% NaCl) supplemented with appropriate antibiotics. *Bacillus* isolates were grown at 30°C in the same medium. All used chemical reagents were of analytical laboratorial grade. Polyphenolic substances were purchased from the following companies located in Germany: Merck KGaA, Darmstadt; Carl Roth GmbH, Karlsruhe; Sigma-Aldrich, Heidelberg and Applichem GmbH, Darmstadt. Additional flavonoids were ordered from Extrasynthese (Lyon, France). Stock solutions of the polyphenols were prepared in DMSO in concentrations of 100 mM.

Isolation of DNA and fosmid library construction

Strain *Bacillus* sp. HH1500 was originally isolated from a soil sample of the botanical garden of the University of Hamburg. DNA from *Bacillus* sp. HH1500 was isolated using the peqGOLD Bacterial DNA Kit (PEQLAB Biotechnologie GmbH, Erlangen, Germany) following the manufacturer protocol. The sample for the construction of the elephant feces library was derived from the Hagenbeck Zoo (Hamburg, Germany). Fresh feces of a healthy six year old female Asian elephant (*Elephas maximus*) named Kandy were taken and stored at -20°C in TE buffer (10 mM TRIS-HC1, 1 mM EDTA, pH 8) containing 30% (v/v) glycerol until DNA extraction. For DNA extraction the QIAamp DNA Stool Mini Kit (Qiagen, Hilden, Germany) was used. The kit was applied according the manufacturer protocol. As recommended the incubation temperature in ASL buffer was increased to 95°C. Isolation of DNA from Elbe river sediment was performed with sediment samples from the tidal flat zone of the river Elbe nearby Gluckstadt (Germany) at low tide (53°44'40" N, 009°, 26' 14" E). Environmental DNA was extracted using the SDS-based DNA extraction method published by Zhou and coworkers (39).

Construction of the genomic and metagenomic libraries in *E. coli* EPI300 cells harboring fosmid *p*CC1FOS was achieved with the CopyControl™ Fosmid Library Production Kit (Epicentre Biotechnologies, Madison, USA) according to the manufacturer protocol using minor modifications as previously published (40). Clones were transferred into 96 well microtitre plates containing 150 μL liquid LB medium with 12.5 μg/mL of chloramphenicol and allowed to grow overnight. Libraries were stored at -70°C after adding 100 μL of 86% glycerol to each microtitre well. The genomic fosmid library of *Bacillus* sp. HH1500 comprised 1,920 clones; a total of 35,000 clones were obtained for the river Elbe sediment library and the elephant feces library encompassed a total of 20,000 clones. All libraries contained fosmids with average

insert sizes of 35 kb.

Molecular cloning strategies

Fragments of *p*CC1FOS fosmids were subcloned into pBluescript II SK+ vector using *Hind*III according to the restriction of the fosmid clones *p*FOS4B2 and *p*FOSI44C11. The resulting plasmids were designated *p*SK4B2 and *p*SK144C11, respectively. Further subcloning of *p*SK144C11-derived fragments was achieved in pTZ19R-Cm with restriction enzymes *Eco*RI and *Pst*I. The obtained clones were designated as *p*TZ144E and *p*TZ144P, respectively. *E. coli* DH5α was transformed with the plasmids by heat shock and the plasmid carrying subclones were identified by blue white screening on LB agar plates containing 10 μM 5-bromo-4-chloro-indolyl-β-D-galactopyranoside (X-Gal) and 400 μM isopropyl-β-D-thiogalactopyranoside (IPTG) after overnight growth. Different clones were analyzed by plasmid purification, followed by enzymatic digestion and agarose gel electrophoresis and/or DNA sequencing.

PCR Amplification of open reading frames (ORFs) was performed with fosmid DNA as a template. The reactions were performed in 30 cycles. To amplify *mgtB* the primers mgt1-*Xho*I-for and mgt1-*Xho*I-rev were used, inserting an *Xho*I endonuclease restriction sites 5' and 3' of the ORF (see TABLE S2). For cloning of *gtf*C primer pair gtf-*Nde*-for and gtf-*Bam*-rev was used, inserting an *Nde*I site including the start codon 5' and a *Bam*HI site 3' of the ORF (TABLE S2). PCR fragments were ligated into *p*Drive using the QIAGEN PCR Cloning Kit (Qiagen, Hilden, Germany) and cloned into *E. coli* DH5α. Resulting clones designated as *p*D*mgtB* and *p*D*gtfC*, respectively, were analyzed for activity in biotransformation and by DNA sequencing for the correct insert. Ligation of *mgtB* and *gtfC* into expression vector *p*ET19b (Novagen, Darmstadt, Germany) was achieved using the inserted endonuclease restriction sites of each ORF. Plasmids containing the correct insert were designated *p*ET19*mgt*B and *p*ET19*gtf*C, respectively. *E. coli* DH5α clones harboring the desired plasmids were detected by direct colony PCR using T7 terminator primer and mgt1-XhoI-for to confirm *mgt*B and T7 terminator primer and gtf Nde-for to verify *gtf*C, respectively. Additionally, the inserts of *p*ET19*mgt*B and *p*ET $19_{gtfC}$ were sequenced using T7 promotor and T7 terminator primers (TABLE S2) to verify the constructs.

Overproduction and purification of enzymes

For overproduction of *deca*-histidin ($His_{10}$-) tagged proteins *E. coli* BL21 (DE3) was transformed with *p*ET19b constructs. An overnight preculture was harvested by centrifugation and 1% was used to inoculate an expression culture. Cells carrying *p*ET19*mgtB* were grown at 22°C until 0.7 $OD_{600}$. The culture was transferred to 17°C and induced by 100 μM IPTG. After 16 h, the culture was harvested by centrifugation at 7.500 g at 4°C. Cells were resuspended in 50 mM phosphate buffer saline (PBS) with 0.3 M NaCl at pH 7.4 and disrupted by ultrasonication with a S2 sonotrode in a UP200S (Hielscher, Teltow, Germany) at a cycle of 0.5 and an amplitude of 75%.

The overproduction of *deca*-histidin-tagged GtfC was induced at 37°C at an $OD_{600}$ of 0.6, with 100 μM IPTG. Cells were then incubated f for four hours, harvested and lysed as stated above for MgtB.

Crude cell extracts were centrifuged at 15.000 g and 4°C to sediment the cell debris. The clarified extracts were loaded on 1 mL HisTrap FF Crude columns using the ÄKTAprime plus system (GE Healthcare). The enzymes were purified according to the manufacturer protocol for gradient elution of His-tagged proteins. Eluted protein solutions were dialyzed twice against 1,000 vol. 50 mM PBS pH 7.4 with 0.3 M NaCl at 4°C. The purification was analyzed on a 12% SDS-PAGE. The concentration of protein was determined by Bradford method using Roti-Quant (Carl Roth GmbH, Karlsruhe, Germany).

Biotransformations and Biocatalyses

For the detection of flavonoid modifications in bacteria a biotransformation approach was used. Cultures were grown in LB medium with appropriate antibiotic overnight. Expression cultures were prepared as stated above for overproduction of enzymes. The cells were sedimented by centrifugation at 4,500 g and resuspended in 50 mM sodium phosphate buffer pH 7 supplemented with 1% (w/v) α-D-glucose. Biotransformations with a final concentration of 100 μM flavonoid inoculated from stock solutions of 100 mM in DMSO, i.e. 0.1%, were incubated in Erlenmeyer flasks at 30°C and 175 rpm up to 24 hours. Samples of 4 mL were withdrawn and acidified with 100 μL 1M $H_3PO_4$ *aq* for extraction in 2 mL ethyl acetate. They were shaken for 1 minute and phase separated by centrifugation at 2,000 g and 4°C. The supernatant was applied in TLC analysis. For quantification, samples of 100 μL were taken and dissolved 1/10 in ethyl acetate/acetic acid 3:1. These acidified ethyl acetate samples were centrifuged at 10,000 g. The supernatant was used for quantitative TLC analysis as stated below.

Fosmid clones were grown in 96 deep well plates overnight. Clones were joined in 96, 48, eight or six clones per pool. The pools were harvested by centrifugation at 4,500 g and resuspended in 50 mL LB medium containing 12.5 μg/mL chloramphenicol, CopyControl™ Autoinduction Solution (Epicentre, Madison, WI) (5 mM arabinose final concentration) and 100 μM of flavonoid for biotransformation. Alternatively to deep well plates, clones were precultured on agar

plates. After overnight incubation the colonies where washed off with 50 mM sodium phosphate buffer pH 7, harvested by centrifugation and resuspended as outlined above. The biotransformations were incubated in 300 mL Erlenmeyer flasks at 30°C with shaking at 175 rpm. Single clones were tested analogously but precultured in 5 mL LB and resuspended in 20 mL biotransformation media in 100 mL flasks. Samples of 4 mL were taken from the reactions after 16, 24 and 48 hours acidified with 40 μL $HCl_{aq}$ and prepared for TLC analysis as stated above. Positive pools were verified in a second biotransformation and then systematically downsized to detect the corresponding hit in a smaller pool until the responsible single clone was identified.

Biocatalytic reactions of 1 mL contained 5 μg of purified His-tagged enzyme and were performed in 50 mM sodium phosphate buffer pH 7 at 37°C. UDP-α-D-glucose or UDP-α-D-galactose was added to final concentrations of 500 μM as donor substrate from 50 mM stock solutions in 50 mM sodium phosphate buffer pH 7. Acceptor substrates were used in concentrations of 100 μM and were added from stock solutions of 100 mM in DMSO leading to a final content of 0.1% in the reaction mixture. The reaction was stopped dissolving 100 μL reaction mixture 1/10 in ethyl acetate/acetic acid 3:1. These samples were used directly for quantitative TLC analysis.

TLC analyses

The supernatant transferred into HPLC flat bottom vials was used for TLC analysis. Samples of 20 μL were applied on 20×10 $cm^2$ (HP)TLC silica 60 $F_{254}$ plates (Merck KGaA, Darmstadt, Germany) versus 200 pmol of reference flavonoids. To avoid carryover of substances, i.e. prevent false positives, samples were spotted with double syringe rinsing in between by the ATS 4. The sampled TLC plates were developed in ethyl acetate/acetic acid/formic acid/water 100:11:11:27 ('Universal Pflanzenlaufmittel') (41). After separation the TLC plates were dried in an oven at 80°C for five minutes. The absorbance of the separated bands was determined densitometrically depending on the absorbance maximum of the applied educt substances at 285 to 370 nm using the deuterium lamp in a TLC Scanner 3 (CAMAG, Muttenz, Switzerland). Subsequently, the substances on developed TLC plates were derivatized by dipping the plates in a methanolic solution of 1% (w/v) diphenyl boric acid β-aminoethyl ester (DPBA) (42) for one second using a Chromatogram Immersion Device (CAMAG, Muttenz, Switzerland) followed by drying the TLC plates in hot air with a fan. After two minutes the bands were visualized at 365 nm with a UV hand lamp and photographed. Alternatively, fluorescence of the bands was determined densitometrically by the TLC Scanner 3 depending on the absorbance maximum of the applied substances at 320 to 370 nm.

Quantification of flavonoids by TLC

To quantify flavonoids in biotransformation and biocatalytic reactions, samples were diluted 1/10 in ethyl acetate/acetic acid 3:1 to stop the reaction. Samples of 20 μL were sprayed by an ATS 4 (CAMAG, Muttenz, Switzerland) on HPTLC silica 60 $F_{254}$ plates (Merck KGaA, Darmstadt, Germany) versus different amounts of respective standard educt and product substances. TLC plates were developed, dried, derivatized and analyzed as stated above. Regression curves were calculated from the peak area of the applied reference substances to determine the amount of produced and residual flavonoids.

HPLC-ESI-MS analysis

HPLC was carried out on a Purospher Star RP-18e 125-4 column (Merck, Darmstadt, Germany), particle size of 3 μm, with a Rheos 2000 pump (Flux Instruments, Suisse) and set pressure limits of 0 bar minimum and a maximum of 400 bar. Injection volumes of 10 μL were separated with solvent A, water supplemented with 0.1% TFA; and solvent B, acetonitrile with 0.1% TFA in following gradient HPLC conditions: From 0 min, 0.6 mL/min 90% A, 10% B; from 14 min, 0.6 mL/min 75% A, 25% B; from 18 min, 0.6 mL/min 5% A, B=95%; from 22 min, 0.6 mL/min 5% A, 95% B; from 22.1 min, 0.6 mL/min, 90% A, 10% B; and from 28.1 min, 0.6 mL/min 90% A, 10% B. Elution was monitored with a Finnigan Surveyor PDA detector and fractions were collected by a HTC PAL autosampler (CTC Analytics). Mass spectrometry (MS) was performed on a Thermo LCQ Deca XP Plus with an ESI interface in positive ionization.

Sequence analysis and Genbank entries

Automated DNA sequencing of small insert plasmids was performed using ABI377 and dye terminator chemistry following the manufacturer's instructions. Large fosmid sequences were established by 454 sequencing technology. The sequences were assembled by using Gap 4 software. ORF finding was performed with Clone manager 9 Professional software. All sequences mentioned here were deposited at GenBank, but were not published before the priority date. The DNA sequences of the *Bacillus* sp. HH1500 16S rRNA gene has the GenBank accession number KC145729. The fosmid derived genes from *B.* sp. HH1500 identified on subclone *p*SK4B2 are *bspA* (JX157885), *mgtB* (JX157886, SEQ

ID NO: 2) and *bspC* (JX157887). The Elbe sediment metagenome derived fosmid subclone *p*SK144C11 comprised genes *esmA* (JX157626), *gtfC* (AGH18139, SEQ ID NO: 1), *esmB* (JX157628), and *esmC* (JX157629).

RESULTS

Screening method: Setup of a TLC-based screening method for the detection of flavonoid-modifying enzyme clones.

"Naturstoffreagenz A". Since it is known that *B. cereus* and *B. subtilis* encode for glycosyltransferases mediating the glucosylation of flavonoids (36), several single bacterial isolates from the applicant's strain collections were initially tested with respect to their flavonoid modifying activities. Biotransformations using whole cells of wild type isolates confirmed the presence of flavonoid modifying enzymes in one of the strains. This strain was originally isolated from a soil sample of the botanical garden in Hamburg and was designated *Bacillus* sp. HH1500. Sequence analysis of a 16S rRNA gene (GenBank entry KC145729) showed a 100% identity to members of the *B. cereus* group (data not shown). In order to use this strain as a positive control, a fosmid library of its genomic DNA in *p*CCIFOS was constructed. The obtained library contained 1,920 clones with an average insert size of 35 kb. Thus, the library encompassed approximately 67 Mb of cloned gDNA hence covering the average size of a genome from *B. cereus* group members about ten times (43). Further, the sensitivity of the (HP)TLC-based assay was verified using a serial dilution of isoquercitrin, the 3-O-β-D-glucoside of quercetin, by spraying 10 μL of 0.78 μM up to 100 μM solutions of isoquercitrin on TLC plates and measuring the absorbance at 365 nm (TABLE 3). In addition, 10 μL of other glycosylated flavonoids were assayed at 10 μM concentrations and could be detected as clear peaks on the absorbance chromatograms (TABLE 3, and data not shown).

Based on the observed sensitivities, a systematic screening scheme was designed. Initially 96 fosmid clones were grown in deep well microtitre plates at 37°C overnight. Cultures were then pooled and following this step, the cells were sedimented by centrifugation and resuspended in fresh LB medium containing the appropriate antibiotics and 100 μM of quercetin as acceptor substrate. After incubation for 16, 24 and 48 hours at 30°C, 4 mL samples of the pooled cultures were withdrawn and extracted with half the volume of ethyl acetate. Of these extracts 20 μL were applied on TLC silica plates and separated using 'Universal Pflanzenlaufmittel' as a solvent. The absorbance of the developed sample lanes was determined densitometrically at 365 nm. Additionally, bands of substrates and modified flavonoids were visualized by staining with 'Naturstoffreagenz A' (42), containing a 1 % solution of diphenylboric acid-β-aminoethylester in methanol; and a 5 % solution of polyethylengycol 4000 in ethanol (available from Carl Roth GmbH, Karlsruhe, Germany). In our hands the sensitivity of the assay was high enough to detect a single flavonoid modifying enzyme clone in a mixture of 96 clones. After the detection of a positive signal, the 96 fosmid clones was divided into pools of 48 to locate the same peak in one of the resulting two half microtitre plates. Following this procedure, the 48 clones were divided to six times eight clones and finally the eight individual clones were analyzed. This strategy was applied successfully to identify six overlapping positive clones in the *Bacillus* sp. HH1500 fosmid library testing all 20 microtitre plates with 1,920 clones, totally.

Of these six fosmid clones, one clone *p*FOS4B2 of approximately 46 kb was subcloned using the *Hind*III restriction site of *p*Bluescript II SK+ vector. The obtained subclones were analyzed using the above-mentioned TLC screening technology. Thereby, a positive subclone designated *p*SK4B2 was identified and completely sequenced (GenBank entry JX157885 - JX157887). Subclone *p*SK4B2 carried an insert of 3,225 bp and encoded for a gene, designated *mgtB*, encoding for a 402 aa protein. The identified ORF was subcloned creating plasmid *pDmgtB* and again assayed for activity. TLC analysis clearly confirmed the glycosylation activity of the MgtB enzyme in this construct as well. The deduced amino acid sequence of MgtB (SEQ ID NO: 8) was highly similar to a predicted *B. thuringiensis* macroside glycosyltransferase (TABLE 1).

**TABLE 1:** Open reading frames (ORF) identified on subclones *p*SK4B2 derived from the active *Bacillus* sp. HH1500 fosmid clone and *p*SK144C11 derived from the river Elbe sediment active fosmid clone.

| Subclone | ORF | AA | Homolog | Coverage (%) | % Identity/Similarity |
|---|---|---|---|---|---|
| *p*SK4B2 | bspA | 221 | putative protein kinase *B. thuringiensis* (ZP04101830) | 100 | 99/99 |
| | mgtB | 402 | macrolide glycosyltransferase *B. thuringiensis* (ZP04071678) | 100 | 98/99 |
| | mgtC | 261 | hypothetical membrane protein *B. thuringiensis* (ZP00741215) | 100 | 99/100 |
| *p*SK144C11 | esmA | 80 | putative UDP-NAc-muramate-L-alanin-ligase *Niabella soli* (ZP09632598) | 99 | 69/80 |

(continued)

| Subclone | ORF | AA | Homolog | Coverage (%) | % Identity/Similarity |
|---|---|---|---|---|---|
| | gtfC | 459 | putative UDP-glucosyltransferase *Fibrisoma limi* (CCH52088) | 92 | 51/71 |
| | esmB | 170 | hypothetical protein *Niastella koreensis* (YP005009630) | 95 | 63/77 |
| | esmC | 150 | putative membrane protein *Solitalea canadensis* (YP006258217) | 98 | 68/81 |

The *mgtB*-surrounding DNA sequences in plasmid *p*SK4B2 represented two truncated genes that consistently were almost identical to genes from *B. thuringiensis* (TABLE 1). This phylogenetic relation was in accordance to the preliminary sequence analysis of the 16S rRNA gene of *Bacillus* sp. HH1500 (see above).

These tests suggested that the screening procedure was suitable for the functional screening of large insert metagenome libraries. For the function-based screening of metagenomes this methodology was termed META: Metagenome Extract TLC Analysis. Although it is not fully automated high-throughput screening (HTS) technology, META allows screening of about 1,200 clones per TLC plate within a time of 48 hours for preculture, biotransformation and analysis. This number of clones appeared to be feasible if the screening was done by single person. Generally, the sampling of about one TLC plate per hour by the ATS 4 is the time limiting step of the method. But this still allows the pooled screening of several plates a day and hence throughput of numerous thousand clones a day by META.

Identification of a novel gylcosyltransferase from a metagenome library

To further apply the screening for enzyme discovery in metagenome libraries, two fosmid libraries constructed in the applicant's laboratory were tested. One library was constructed from DNA isolated from river Elbe sediment the other from isolated DNA out of fresh elephant feces. Altogether both libraries encompassed approximately 50,000 clones with an average insert size of 35 kb. Both libraries were screened using quercetin as a substrate. Using the described strategy one positive microtitre plate pool in the river Elbe-sediment-library was discovered. Further screening of this pool resulted in the identification of a single positive fosmid clone designated *p*FOS144C11. Biotransformations of quercetin (Q) with 48 clone pools presented one product peak (P2) by TLC separation with an Rf value comparable to that of quercitrin, the quercetin-3-O-β-L-rhamnoside. A second peak (P3) with a Rf value higher than the available reference quercetin glycones was observed in conversions with the six-clone-pool and the single fosmid clone, respectively. Clone *p*FOS 144C11 carried a fosmid of approximately 40 kb. Subsequent restriction fragment subcloning into *p*Bluescript II SK+ with *Hind*III yielded in the identification of the positive *E. coli* DH5α subclone *p*SK144C11. However, biotransformations with *p*SK144C11 showed two product peaks, a major one (P2) with an Rf value comparable to that of quercitrin and a minor one (P1) similar to isoquercitrin. The subclone *p*SK144C11 still had an insert of approximately 8.5 kb size. Further sequencing and subcloning of *p*SKI44C11 finally identified the gene putatively responsible for the modifications which was designated *gtfC*. The deduced 459 amino acid sequence (see SEQ ID NO: 7) of the corresponding enzyme revealed motif similarities to UDP-glucuronosyl/UDP-glucosyltransferases. GtfC (SEQ ID NO: 7) showed a similarity of 71% to the putative glycosyltransferase of the Gram-negative bacterium *Fibrisoma limi* covering 92% of the protein (TABLE 1). Further cloning of the *gtfC* ORF into *p*Drive vector and biotransformation with *E. coli* DH5α carrying the respective construct *p*D*gtfC* confirmed the flavonoid-modifying activity of GtfC.

In summary, these results demonstrated that the developed screening procedure META is sufficiently sensitive to allow the identification of large insert clones from individual bacterial genomes (i.e. *Bacillus* sp. HH1500) and complex metagenome libraries (i.e. the river Elbe sediment library) showing flavonoid-modifying activities.

Sequence based classification of MgtB and GtfC

To analyze the affiliation of MgtB and GtfC, a phylogenetic tree using the MEGA version 5 software (44) was calculated. The amino acid (aa) sequences of MgtB (SEQ ID NO: 8) and GtfC (SEQ ID NO: 7), and their closest sequence-based relatives determined by pBlast were aligned by ClustalW. Additionally, the sequences of the actually published prokaryotic flavonoid active GTs were aligned and finally as an outer group two eukaryotic enzymes, the flavonoid glucosyltransferase UGT85H2 from *Medicago truncatula* and the flavonoid rhamnosyltransferase UGT78D1 from *Arabidopsis thaliana* (45-46, 53). Thereof a neighborjoining tree with 100 bootstraps was computed. As expected, MgtB from *Bacillus* sp. HH1500 clustered with other MGTs from the *B. cereus* group. At time of writing, the MGT of *B. thuringiensis* IBL 200 and the MGT of *B. cereus* G9842 turned out to be the closest relatives with an aa identity to MgtB of

98 % each. Both MGTs were annotated as predicted enzymes and no substrate data were available. From the MGT cluster five other enzymes already were reported to mediate the glucosylation of flavonoids. Three of them BcGT-1 the nearest relative reported to be flavonoid active, BcGT-4, and BcGT-3 all originated from *B. cereus* ATCC10987 (47-49). Another flavonoid active MGT, designated BsGT-3, originates from *B. subtilis* strain 168 (36). The remaining flavonoid active MGT is the well-studied OleD from *Streptomyces antibioticus* (50, 51). GtfC was located in a distinct cluster of UGTs and appeared to be somewhat related to hypothetical enzymes from *Cytophagaceae* bacteria as *Dyadobacter fermentans* and *Fibrisoma limi*. Within this cluster only the UGT XcGT-2 is known to accept flavonoid substrates (38). Interestingly, rhamnosyltransferases like BSIG 4748 from *Bacteroides* sp. 116 and RtfA from *Mycobacterium avium* phylogenetically also show affiliation to this cluster but forming a separate branch.

To further characterize the identified metagenome-derived GTs, the aa residues of the C-terminal donor binding regions were compared to the motifs of the closest relatives and the known flavonoid active GTs. Here, the Rossmann fold $\alpha/\beta/\alpha$ subdomain, the conserved donor-binding region of UGTs, is located (52). Plant UDP-glycosyltransferases like UGT85H2 and UGT78D1 exhibit a highly conserved motif in this region which is termed the (Plant Secondary Product Glycosyltransferase) PSPG motif (45, 53-54). By alignment key aa known to be of importance for NDP-sugar binding could be identified. While MgtB revealed a clear UDP-hexose binding motif consisting of highly conserved Gln289 and Glu310 residues for ribose binding and a conserved DQ, GtfC lacked this motif (45, 55, 56). Instead, GtfC presented typical residues Phe336 and Leu357 for deoxy ribose nucleotide utilization (57). Moreover the pyrophosphate binding sites in the MgtB aa sequence could be identified. However, GtfC does not possess these conserved phosphate binding residues suggesting that GtfC and related enzymes have another donor binding mode. In this context GtfC seemed to belong to a novel enzyme class underlining the low level of sequence homology.

Overexpression and glycosylation patterns of MgtB and GtfC

To further characterize the novel enzymes and verify their functions, MgtB and GtfC were overexpressed and purified as His-tagged proteins in *E. coli* BL21 (DE3). Both genes *mgtB* and *gtfC* were ligated into the expression vector pET19b. The recombinant enzymes containing N-terminal His$_{10}$-tags were purified by Ni-affinity chromatography in native conditions and gradient elution. MgtB could be purified with more than 5 mg/g cell pellet (wet weight). The maximum yield of GtfC was 3 mg/g of cell pellet. The molecular weights of the proteins were verified by SDS-PAGE analysis in denaturing conditions according to Laemmli. After Coomassie-staining, His$_{10}$-MgtB was visible as a single band with a MW of approximately 50 kDa on a 12% SDS-PAGE. This was in accordance with the calculated molecular weight (MW) of 51.2 kDa including the N-terminal His-tag. His$_{40}$-GtfC revealed a MW of about 55 kDa on a 12% SDS-PAGE which was in well accordance to the calculated MW of 54.7 kDa including the N-terminal His-tag. While virtually no additional bands were visible on SDS-PAGEs with purified recombinant MgtB protein, some minor contaminating bands were still visible on the SDS-PAGE loaded with purified GtfC. In summary both proteins could be purified to allow further biochemical characterization.

The purified His$_{10}$-MgtB protein was able to use UDP-$\alpha$-D-glucose as a donor substrate. The recombinant enzyme catalyzed the transfer of $\alpha$-D-glucose residues to various polyphenols. Biocatalytic reactions were performed with 500 $\mu$M UDP-$\alpha$-D-glucose as donor and 100 $\mu$M of acceptor substrate. The following flavonoids served as acceptor substrates and were modified with high yields: Luteolin, quercetin, kaempferol, tiliroside, naringenin, genistein (TABLE 2).

**TABLE 2:** Flavonoid substrates converted by recombinant MgtB in bioassays. Reactions of 1 mL were carried out at 37 °C for 2 hours in triplicate with 500 $\mu$M UDP-glucose, 100 $\mu$M of the respective flavonoid and 5 $\mu$g/mL of purified and recombinant MgtB.

| Substrate | | Conversion (%) | Rf value[a] | Product(s)[b] |
|---|---|---|---|---|
| Quercetin | 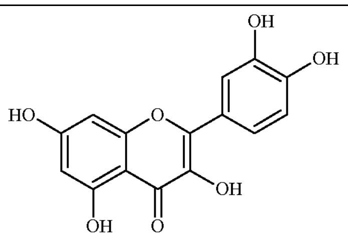 | ~100% | 0.79 | - |
| | | | **0.64** | **Isoquercitrin** |
| | | | 0.27 | - |
| | | | 0.25 | - |

(continued)

| Substrate | | Conversion (%) | Rf value[a] | Product(s)[b] |
|---|---|---|---|---|
| Kaempferol | 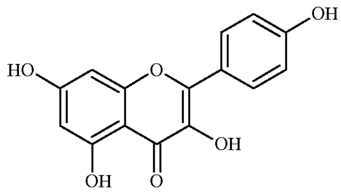 | ~100% | **0.74**<br>0.35 | **Astragalin**<br>- |
| Luteolin | 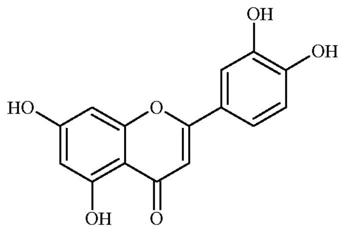 | 82% | **0.65**<br>0.32 | **Cynaroside**<br>-3',7-di-O-Glc |
| Naringenin | 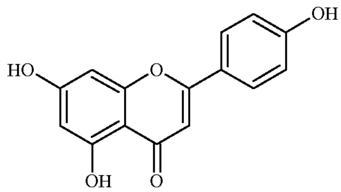 | 52% | 0.76 | Prunin |
| Genistein | 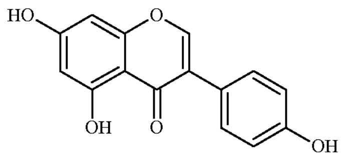 | 72% | 0.69 | Genistin |
| Tiliroside | 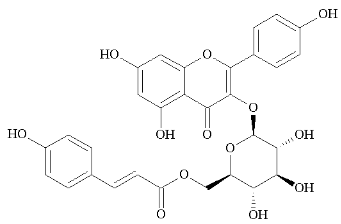 | 83% | 0.54 | - |

[a]Rf values and products in **bold** indicate the main product of the biocatalytic reactions.
[b]Products symbolized by "-" were not specified due to unavailable reference substances.

Thereby flavonols turned out to be the best acceptor molecules. Generally, the conversion during a two-hour assay ranged from 52% for naringenin and approximately 100% for quercetin and kaempferol. Interestingly, in the presence of quercetin and kaempferol no residual educts could be monitored by HPTLC analysis. The specific educts and their observed glycones of the biocatalytic reactions are summarized in TABLE 2 together with the respective Rf values. MgtB favored the glucosylation at the C3 hydroxy group if accessible like in the aglycone flavonols quercetin and kaempferol. Further, the C7-OH was attacked and glucosylated by the enzyme which could be shown for the flavone luteolin but also the flavanone naringenin and the isoflavone genistein (TABLE 2). MgtB glucosylated luteolin also at the C3' hydroxy group forming the 3',7-di-O-glucoside of luteolin if the C7-OH was glucosylated previously. MgtB also catalyzed the conversion of the kaempferol derivative tiliroside, the kaempferol-3-O-6"-coumaroyl-glucoside. One glucosylated product with a Rf values of 0.54 was detected. The chalcone xanthohumol and the stilbene *t*-resveratrol were tested in biotransformation reactions with *E. coli* expressing *mgtB* but conversions were not quantified (data not shown). Xanthohumol yielded three detectable products whereas the biotransformation of *t*-resveratrol yielded one observed product by absorbance TLC analysis.

Tests with recombinant and purified GtfC using UDP-α-D-glucose and UDP-α-D-galactose and quercetin as acceptor molecule suggested that *d*TDP-activated sugar moieties were transferred by this enzyme. This finding was confirmed by HPLC-ESI-MS analyses of biotransformation assays (see following paragraph). Unfortunately, deoxy-ribose nucleotide activated hexoses e.g. *d*TDP-rhamnoside were commercially not available to further analyze the obtained reaction products in more detail (58).

Biotransformations with the *E. coli* strain expressing GtfC and using various polyphenols as substrates yielded in conversions ranging from 52% for xanthohumol up to almost 100% turnover for most flavonols tested (TABLE 3).

**TABLE 3:** Flavonoid substrates and products of biotransformation assays with recombinant GtfC. Quantification of the reaction was performed as described herein. Triplicate reactions of 50 mL were performed in 50 mM sodium phosphate buffer (PB) pH 7.0 containing 1% (w/v) glucose and 200 μM of flavonoid at 30 °C.

| | Substrate | Conversion (%) | Rf value[a] | Product(s)[b] |
|---|---|---|---|---|
| Luteolin | 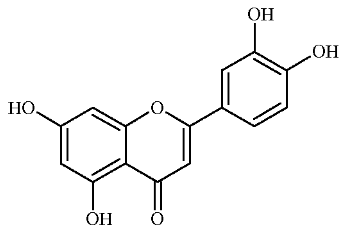 | 86 | 0.81 | - |
| | | | **0.73** | - |
| | | | 0.68 | - |
| | | | 0.58 | - |
| Quercetin | 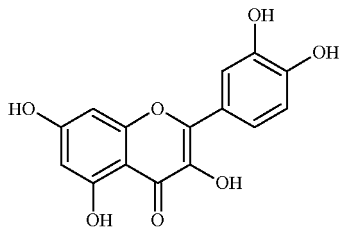 | ~100 | 0.82 | - |
| | | | **0.75** | **Quercitrin** |
| | | | 0.64 | Isoquercitrin |
| Kaempferol | 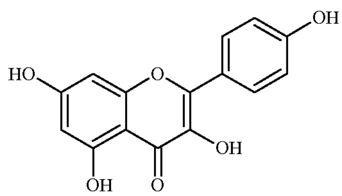 | ~100 | 0.85 | - |
| | | | **0.80** | - |
| | | | 0,68 | Astragalin- |
| Naringenin | 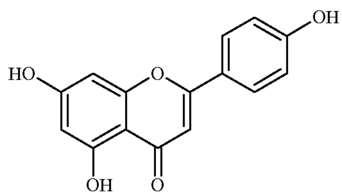 | 76 | 0.87 | - |
| | | | 0.84 | - |
| | | | **0.77** | **Prunin** |
| Genistein | 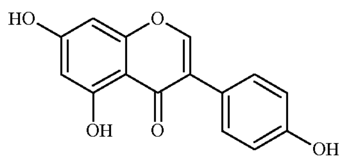 | 68 | 0.83 | - |
| | | | **0.76** | - |
| | | | 0.68 | Genistin |
| *t*-Resveratrol | 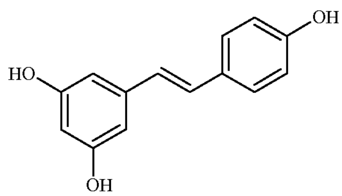 | 96 | 0.83 | - |
| | | | **0.77** | - |
| | | | 0.64 | - |
| | | | 0.58 | - |
| | | | 0.51 | - |
| | | | 0.46 | - |

(continued)

| | Substrate | Conversion (%) | Rf value[a] | Product(s)[b] |
|---|---|---|---|---|
| Xanthohumol | 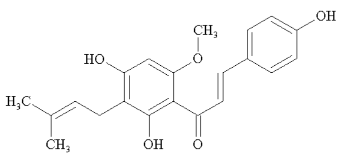 | 52 | 0.85<br>0.48 | -<br>- |

[a]Rf values and products in **bold** indicate the main product of the biotransformation reactions.
[b]Products symbolized by "-" were not specified due to unavailable reference substances

Quercetin was transformed almost completely after four-hour biotransformations and yielded three detectable products (P1-P3). To further characterize these products UV absorbance spectra were recorded and compared to the reference glycones of quercetin isoquercitrin and quercitrin (59). P1 revealed an Rf value identical to the value of isoquercitrin. Further the UV absorbance spectrum of P1 matched the spectrum of isoquercitrin. P2 revealed an Rf value identical to the one known for quercitrin. P2 also exhibited the same UV absorbance spectrum as quercitrin. P3 revealed an Rf value of 0.82, which clearly differed from the RF values of known and available quercetin glycones. Compared to isoquercitrin, P3 showed a similar hypsochromic shift of band I to a $\lambda_{max}$ of 363 nm; however it revealed a less hypsochromic shift in band II of only 5 nm to 272 nm with a shoulder at 280 nm. It is further notable that the HPLC-ESI-MS analysis of biotransformation products of quercetin consistently identified three distinct reaction products. P1 had a RT of 17.93 min in the HPLC analysis and revealed a molecular mass of 464 u, which is equivalent to isoquercitrin. P2 revealed a RT of 18.06 min and had a molecular mass of 448 u. This mass corresponds well with the molecular mass of quercitrin. Finally, P3 with a RT of 18.31 min revealed a molecular mass of 446 u indicating the formation of a novel not further characterized quercetin glycoside.

Glycosylation patterns of GtfC on quercetin suggested a preference to act on the C3 hydroxy group mediating the transfer of different sugar residues. However, if a C3 OH-group was not available, GtfC efficiently catalyzed the glycosylation of other positions. Flavones lacking the hydroxy function at C3 were converted depending on the availability of other hydroxy groups. Pratol possessing only a single free C7-hydroxy group was converted weakly and resulted in a single detectable product. Further the biotransformation of 3',4'-dihydroxyflavone yielded three detectable glycones and 5-methoxy-eupatorin yielded two products (data not shown); the biotransformation of the mono 4'-hydroxyflavanone yielded one glycosylated product and the glycosylation of naringenin yielded two products. The major biotransformation product of naringenin revealed the same Rf value and absorbance spectrum as prunin, the naringenin-7-O-glucoside (TABLE 3). The second naringenin glycone could not be further specified due to the lack of commercially available reference substances. Altogether these results suggested that GtfC acts on the C3, C3', C4' and C7 hydroxy groups of the flavonoid backbone.

In summary these data demonstrated that MgtB and GtfC possess interesting biocatalytic properties. While MgtB specifically mediated the transfer of glucose residues, GtfC transferred different hexose moieties. MgtB was capable to catalyze the glucosylation of already glycosylated flavonoids to form di-glycosides (e.g. formation of luteolin-3',7-di-O-glucoside) and even tiliroside to generate novel glucosides not available from natural resources. In contrast, the glycosylation pattern of GtfC suggested the transfer of single sugar residues to only aglycone flavonoid forms. Interestingly, GtfC seemed to be very variable concerning its activity at various positions on the flavonoid backbone. This may lead to the formation of truly novel flavonoids naturally not available. Hence both enzymes might be helpful in the generation of new natural compounds.

Using a novel screening technology, a macroside glycosyltransferase MgtB from a soil isolate (i.e. *Bacillus* sp. HH1500) has been identified. A fosmid library established with DNA from this strain, which had been isolated from the local botanical garden, only recently, was initially used to develop and verify the outlined screening technology; and using the novel screening technology, MgtB was quickly identified from a pool of almost 2,000 clones. Isolation and purification of recombinant MgtB revealed a novel MGT. MgtB shared 89% aa identity with BcGT-1 from *B. cereus* ATCC 10987, the closest relative published to act on flavonoids. BcGT-1 was reported to catalyze the glucosylation of flavones, flavonols, flavanones and isoflavones (47). On flavonols BcGT-1 acted on C3-, C7- and C4'- hydroxy groups creating triglucosides of kaempferol (48). In contrast biocatalyses of kaempferol with MgtB yielded just two detectable glucosylated products. Instead reactions with quercetin resulted in three detectable glycones. These data suggested that MgtB acted at the C3' OH-group. This hypothesis was also was supported by the observation that recombinant MgtB converted luteolin to luteolin-3',7-di-O-glucoside as a byproduct. These results were in accordance with the glucosylation pattern of BcGT-3 yet another MGT from *B. cereus* ATCC10987 (49). Interestingly, BcGT-3 shares only 40% aa identity with MgtB but both enzymes act on the same flavonoids forming di-glucosides from flavones and flavonols at the same positions and only mono-glucosides from naringenin. The most spectacular conversion observed for MgtB was that of

tiliroside. The product is likely to be the 7-O-glucoside taking the glycosylation pattern of MgtB into account. Tiliroside glycosides yet were not reported in scientific literature. This raises the possibility of the generation of new natural compounds. The natural substrates of *Bacillus* MGTs still have not been reported. Other MGTs like OleD usually detoxify macroside antibiotics but often possess broad acceptor tolerance (35, 60).

The metagenome-derived GtfC turned out to be a completely novel enzyme. Only seven flavonoid-active UGTs have been reported so far that originate from five different prokaryotes (35, 36, 38, 47, 49). Without XcGT-2 from Gram-negative *X. campestris* ATCC 33913 all remaining are MGT enzymes from Gram-positive Bacilli and Streptomycetes. MGTs play an important role in xenobiotic defense mechanisms of prokaryotes and thus show broad acceptor specificities (55, 60). This also applies for eukaryotic UGTs pointing to a biological principle of detoxification (61). To our knowledge GtfC is the first metagenome-derived GT acting on flavonoids. Moreover, it is also the first bacterial enzyme reported to transfer various *d*TDP activated hexose sugars to polyphenols (see below) in contrast to usually stringent donor specificities like Gtfs (57). With respect to the notion that many NDP-sugars in prokaryotes are *d*TDP and not UDP activated, GtfC might be a promising biocatalyst in glycodiversification approaches (58, 62, 63). GtfC is similar to predicted GTs from *Cytophagaceae* bacteria (64-66). These Gram-negative bacteria have large genomes suggesting extensive secondary metabolic pathways and they are well known for the presence of resistance mechanism to antibiotics as trimethoprim and vancomycin (67, 68). As commonly known glycosylation of xenobiotics is a ubiquitous detoxification process in all kingdoms of life. The phylogenetically divers members of *Cytophagaceae* have only recently become an object of research and a concrete estimation about the phylogenetic wideness of this family and exact taxonomic ranking still remain unclear (65, 69). Thus, the identification of the metagenome-derived GtfC and its partial characterization suggest that this group of microorganisms is perhaps highly promising resource for novel GTs and also other enzymes.

A ClustalW alignment of the donor-binding region of GtfC suggested the activated donor substrates are of deoxy-thymidine nucleoside origin. GtfC possesses the typical aa residues Phe336 for thymine base stacking and hydrophobic Leu357 for deoxy-ribose fitting (57). Concerning the donor binding of GTs GtfC appears to not exhibit the known aa residues for pyrophosphate binding. Instead of the conserved residue His/Arg in the up to date solved protein structures GtfC contains an Asn at the aa position 349 (52, 70). This applies also for the nearest GtfC relatives Dfer1940, UGT of *F. limi* BUZ 3 and Slin3970 as well as the NGTs RebG and BSIG4748. Further, GtfC does not show the conserved Ser/Thr residue responsible for α-phosphate binding. Instead the Gly354 appears to be of importance for the α-phosphate binding similar to the OleD transferase (55).

The assumption of *d*TDP activated co-substrates used by GtfC was supported by the observation that glucose, rhamnose and a third sugar residue with molecular weight of 446 were transferred by GtfC in biotransformations using intact *E. coli* cells. Besides, biocatalytic approaches with purified GtfC and either UDP-α-D-glucose or -galactose as donor substrates failed. In bacteria, the activated sugars, *d*TDP-α-D-glucose, -4-keto-6-deoxy-α-D-glucose or -4-keto-β-L-rhamnose, and -β-L-rhamnose are part of the *d*TDP-sugar biosynthesis pathway and are present in *E. coli* (71). Moreover, levels of *d*TDP-sugars are allosterically regulated by *d*TDP-rhamnose levels through activity of RmlA (72).

Four additional glycosyltransferases were identified and designated MgtT, MgtC, MgtS and MgtW.

MgtT:

397 aa (SEQ ID NO: 11), gene 1194 bp (SEQ ID NO: 5), from *Bacillus* sp. BCHH1500; 99% aa identity to MGT from *B. cereus* B4264 (YP002367512)
Reaction in biotransformation (whole cell catalysis) with *E. coli* DH5α pDrive::mgtT shown for e.g. 4-Methylumbelliferone (4-MU, 7-Hydroxy-4-methylcoumarin), phloretin, homoeriodytiol, naringenin, *et al.*

MgtC:

402 aa (SEQ ID NO: 9), gene 1209 bp (SEQ ID NO: 3), from *Bacillus* sp. BCG+1; 95% aa identity to MGT aus *B. cereus* ATCC10987 (NP978481)
Reaction in biotransformation (whole cell catalysis) with *E. coli* DH5α pDrive::mgtC shown for apigenin, luteolin; quercetin, naringenin, homoeriodytiol, phloretin, noreugenin, *et al.*

Exemplary reaction scheme:

UDP-α-D-Glucose + flavonoid –> Flavonoid-β-D-glucoside + UDP

MgtS:

392 aa (SEQ ID NO: 10), gene 1179 bp (SEQ ID NO: 4), from *Bacillus subtilis* BSHH14 99% aa identity zu MGT YjiC aus *B. subtilis* (YP007533161)
Reaction in biotransformation (whole cell catalysis) with *E. coli* BL21(DE3) *p*ET19b::*mgtS* and biocatalysis with enzyme shown for phloretin, homoeriodytiol, naringenin, apigenin, luteolin; quercetin, 4-Methylumbelliferon, noreugenin, *et al.*

Exemplary reaction scheme:

UDP-α-D-Glucose + polyphenol –> polyphenol-β-D-glucoside + UDP

MgtW:

402 aa (SEQ ID NO: 12), gene 1209 bp (SEQ ID NO:6), from *Bacillus* sp. BCHH03 99% aa identity to MGT from *B. weihenstephanensis* KBAB4 (YP001644794)
Reaction in biotransformation (whole cell catalysis) with *E. coli* DH5α pDrive::*mgtW* shown for quercetin, phloretin, homoeriodyctiol, *et al.*

Exemplary reaction scheme:

UDP-α-D-Glucose + quercetin –> quercetin 3-O-β-D-glucoside + UDP

Overview of sequences (aa = number of amino acids, bp = number of base pairs, PRT = protein, nt = number of nucleotides):

| SEQ ID NO: | Type | aa | bp/nt | description |
|---|---|---|---|---|
| 1 | DNA | | 1380 | *gtfC* gene |
| 2 | DNA | | 1209 | *mgtB* gene |
| 3 | DNA | | 1209 | *mgtC* gene |
| 4 | DNA | | 1179 | *mgtS* gene |
| 5 | DNA | | 1194 | *mgtT* gene |
| 6 | DNA | | 1209 | *mgtW* gene |
| 7 | PRT | 459 | | GtfC protein |
| 8 | PRT | 402 | | MgtB protein |
| 9 | PRT | 402 | | MgtC protein |
| 10 | PRT | 392 | | MgtS protein |
| 11 | PRT | 397 | | MgtT protein |
| 12 | PRT | 402 | | MgtW protein |
| 13 | DNA | | 20 | cfn_GT-1for |
| 14 | DNA | | 19 | cfn_GT-for |
| 15 | DNA | | 29 | cfn_GT-rev |
| 16 | DNA | | 29 | gtf-*Nde*-for |
| 17 | DNA | | 27 | gtf-*Bam*-rev |
| 18 | DNA | | 22 | mgt-1-*Xho*I-for |
| 19 | DNA | | 25 | mgt-1-*Xho*I-rev |
| 20 | DNA | | 17 | T3 promoter |
| 21 | DNA | | 19 | T7 promoter |

(continued)

| SEQ ID NO: | Type | aa | bp/nt | description |
|---|---|---|---|---|
| 22 | DNA | | 19 | T7 terminator |

References

19. Altschul, S.F., Gish, W., Miller, W., Myers, E.W. & Lipman, D.J. (1990) "Basic local alignment search tool." J Mol Biol 215:403-410

20. Henikoff, S. and Henikoff, J., Amino acid substitution matrices from protein blocks. Proc Natl Acad Sci USA 89: 10915-10919, 1992

21. Ververidis, F., Trantas, E., Douglas, C., Vollmer, G., Kretzschmar, G., and Panopoulos, N. 2007. Biotechnology of flavonoids and other phenylpropanoid-derived natural products. Part II: Reconstruction of multienzyme pathways in plants and microbes. Biotechnol J 2:1235-1249.

22. Schütz, K., Muks, E., Carle, R., and Schieber, A. 2006. Quantitative determination of phenolic compounds in artichoke-based dietary supplements and pharmaceuticals by high-performance liquid chromatography. J Agric Food Chem 54:8812-8817.

23. Leonard, E., Yan, Y., Fowler, Z. L., Li, Z., Lim, C. G., Lim, K. H., and Koffas, M. A. 2008. Strain improvement of recombinant Escherichia coli for efficient production of plant flavonoids. Mol Pharm 5:257-265.

24. Wang, C., Meek, D. J., Panchal, P., Boruvka, N., Archibald, F. S., Driscoll, B. T., and Charles, T. C. 2006. Isolation of poly-3-hydroxybutyrate metabolism genes from complex microbial communities by phenotypic complementation of bacterial mutants. Appl Environ Microbiol 72:384-391.

25. Manach, C., Scalbert, A., Morand, C., Remesy, C., and Jimenez, L. 2004. Polyphenols: food sources and bioavailability. Am J Clin Nutr 79:727-747.

26. Das, S., and Rosazza, J. P. 2006. Microbial and enzymatic transformations of flavonoids. J Nat Prod 69:499-508.

27. Graefe, E. U., Wittig, J., Mueller, S., Riethling, A. K., Uehleke, B., Drewelow, B., Pforte, H., Jacobasch, G., Derendorf, H., and Veit, M. 2001. Pharmacokinetics and bioavailability of quercetin glycosides in humans. J Clin Pharmacol 41:492-499.

28. Kren, V., and Martinkova, L. 2001. Glycosides in medicine:The role of glycosidic residue in biological activity. Curr Med Chem 8:1303-28.

29. Coutinho, P. M., Deleury, E., Davies, G. J., and Henrissat, B. 2003. An evolving hierarchical family classification for glycosyltransferases. J Mol Biol 328:307-317.

30. Bowles, D., Lim, E. K., Poppenberger, B., and Vaistij, F. E. 2006. Glycosyltransferases of lipophilic small molecules. Annu Rev Plant Biol 57:567-597.

31. Mackenzie, P. I., Owens, I. S., Burchell, B., Bock, K. W., Bairoch, A., Belanger, A., Fournel-Gigleux, S., Green, M., Hum, D. W., Iyanagi, T., Lancet, D., Louisot, P., Magdalou, J., Chowdhury, J. R., Ritter, J. K., Schachter, H., Tephly, T. R., Tipton, K. F., and Nebert, D. W. 1997. The UDP glycosyltransferase gene superfamily: recommended nomenclature update based on evolutionary divergence. Pharmacogenetics 7:255-269.

32. Lairson, L. L., Henrissat, B., Davies, G. J., and Withers, S. G. 2008. Glycosyltransferases: structures, functions, and mechanisms. Annu Rev Biochem 77:521-555.

33. Osmani, S. A., Bak, S. r., and Moller, B. L. 2009. Substrate specificity of plant UDP-dependent glycosyltransferases predicted from crystal structures and homology modeling. Phytochemistry 70:325-347.

34. Breton, C., Snajdrova, L., Jeanneau, C., Koca, J., and Imberty, A. 2006. Structures and mechanisms of glycosyltransferases. Glycobiology 16:29R-37.

35. Yang, M., Proctor, M. R., Bolam, D. N., Errey, J. C., Field, R. A., Gilbert, H. J., and Davis, B. G. 2005. Probing the breadth of macrolide glycosyltransferases: in vitro remodeling of a polyketide antibiotic creates active bacterial uptake and enhances potency. J Am Chem Soc 127:9336-9337.

36. Jeon, Y., Kim, B., Kim, J., Cheong, Y., and Ahn, J.-H. 2009. Enzymatic Glycosylation of Phenolic Compounds Using BsGT-3. Journal of the Korean Society for Appl Biol Chem 52:98-101.

37. Rao, K. V., and Weisner, N. T. 1981. Microbial Transformation of Quercetin by Bacillus cereus. Appl Environ Microbiol 42:450-452.

38. Kim, H. J., Kim, B. G., Kim, J. A., Park, Y., Lee, Y. J., Lim, Y., and Ahn, J. H. 2007. Glycosylation of flavonoids with E. coli expressing glycosyltransferase from Xanthomonas campestris. J Microbiol Biotechnol 17:539-542.

39. Zhou, J., Bruns, M. A., and Tiedje, J. M. 1996. DNA recovery from soils of diverse composition. Appl Environ Microbiol 62:316-322.

40. Ilmberger, N., Meske, D., Juergensen, J., Schulte, M., Barthen, P., Rabausch, U., Angelov, A., Mientus, M.,

Liebl, W., Schmitz, R. A., and Streit, W. R. 2012. Metagenomic cellulases highly tolerant towards the presence of ionic liquids--linking thermostability and halotolerance. Appl Microbiol Biotechnol 95:135-146.

41. Wagner, H., Bladt, S., Zgainski, E. M. 1983. Drogenanalyse, Dünnschichtchromatographische Analyse von Arzneidrogen. Springer Publisher, Berlin Heidelberg New York.

42. Neu, R. 1957. Chelate von Diarylborsäuren mit aliphatischen Oxyalkylaminen als Reagenzien fur den Nachweis von Oxyphenyl-benzo-γ-pyronen. Naturwissenschaften 44:181-182.

43. Tourasse, N. J., Helgason, E., Økstad, O. A., Hegna, I. K., and KolstØ, A. B. 2006. The Bacillus cereus group: novel aspects of population structure and genome dynamics. Appl Microbiol 101:579-593.

44. Tamura K, P. D., Peterson N, Stecher G, Nei M, and Kumar S. 2011. MEGA5: Molecular Evolutionary Genetics Analysis using Maximum Likelihood, Evolutionary Distance, and Maximum Parsimony Methods. Mol Biol Evol 28:2731-2739.

45. Li, L., Modolo, L. V., Escamilla-Trevino, L. L., Achnine, L., Dixon, R. A., and Wang, X. 2007. Crystal structure of Medicago truncatula UGT85H2--insights into the structural basis of a multifunctional (iso)flavonoid glycosyltransferase. J Mol Biol 370:951-963.

46. Jones, P., Messner, B., Nakajima, J.-I., Schäffner, A. R., and Saito, K. 2003. UGT73C6 and UGT78D1, Glycosyltransferases Involved in Flavonol Glycoside Biosynthesis in Arabidopsis thaliana. J Biol Chem 278:43910-43918.

47. Ko, J. H., Gyu Kim, B., and Joong-Hoon, A. 2006. Glycosylation of flavonoids with a glycosyltransferase from Bacillus cereus. FEMS Microbiol Lett 258:263-268.

48. Jung, N. R., Joe, E. J., Kim, B. G., Ahn, B. C., Park, J. C., Chong, Y., and Ahn, J. H. 2010. Change of Bacillus cereus flavonoid O-triglucosyltransferase into flavonoid O-monoglucosyltransferase by error-prone polymerase chain reaction. J Microbiol Biotechnol 20:1393-1396.

49. Ahn, B. C., Kim, B. G., Jeon, Y. M., Lee, E. J., Lim, Y., and Ahn, J. H. 2009. Formation of Flavone Di-O-Glucosides Using a Glycosyltransferase from Bacillus cereus. J Microbiol Biotechnol 19:387-390.

50. Choi, S. H., Ryu, M., Yoon, Y. J., Kim, D. M., and Lee, E. Y. 2012. Glycosylation of various flavonoids by recombinant oleandomycin glycosyltransferase from Streptomyces antibioticus in batch and repeated batch modes. Biotechnol Lett 34:499-505.

51. Williams, G. J., Zhang, C., and Thorson, J. S. 2007. Expanding the promiscuity of a natural-product glycosyltransferase by directed evolution. Nat Chem Biol 3:657-662.

52. Hu, Y., and Walker, S. 2002. Remarkable structural similarities between diverse glycosyltransferases. Chem Biol 9:1287-1296.

53. Hughes, J., and Hughes, M. A. 1994. Multiple secondary plant product UDP-glucose glucosyltransferase genes expressed in cassava (Manihot esculenta Crantz) cotyledons. Mitochondrial DNA 5:41 - 49.

54. Paquette, S., Moller, B. L., and Bak, S. 2003. On the origin of family 1 plant glycosyltransferases. Phytochemistry 62:399-413.

55. Bolam, D. N., Roberts, S., Proctor, M. R., Turkenburg, J. P., Dodson, E. J., Martinez-Fleites, C., Yang, M., Davis, B. G., Davies, G. J., and Gilbert, H. J. 2007. The crystal structure of two macrolide glycosyltransferases provides a blueprint for host cell antibiotic immunity. Proc Natl Acad Sci U S A 104:5336-5341.

56. Offen, W., Martinez-Fleites, C., Yang, M., Kiat-Lim, E., Davis, B. G., Tarling, C. A., Ford, C. M., Bowles, D. J., and Davies, G. J. 2006. Structure of a flavonoid glucosyltransferase reveals the basis for plant natural product modification. Embo J 25:1396-1405.

57. Mulichak, A. M., Lu, W., Losey, H. C., Walsh, C. T., and Garavito, R. M. 2004. Crystal structure of vancosaminyltransferase GtfD from the vancomycin biosynthetic pathway: interactions with acceptor and nucleotide ligands. Biochemistry 43:5170-5180.

58. Lim, E.-K., Ashford, D. A., and Bowles, D. J. 2006. The Synthesis of Small-Molecule Rhamnosides through the Rational Design of a Whole-Cell Biocatalysis System. ChemBioChem 7:1181-1185.

59. Mabry, T. J., Markham, K. R., and Thomas, M. B. 1970. The systematic identification of flavonoids. New York. Springer-Verlag.

60. Gantt, R. W., Goff, R. D., Williams, G. J., and Thorson, J. S. 2008. Probing the aglycon promiscuity of an engineered glycosyltransferase. Angew Chem Int Ed Engl 47:8889-8892.

61. Vogt, T., and Jones, P. 2000. Glycosyltransferases in plant natural product synthesis: characterization of a supergene family. Trends Plant Sci 5:380-386.

62. Williams, G. J., Gantt, R. W., and Thorson, J. S. 2008. The impact of enzyme engineering upon natural product glycodiversification. Curr Opin Chem Biol 12:556-564.

63. Yoon, J. A., Kim, B. G., Lee, W. J., Lim, Y., Chong, Y., and Ahn, J. H. 2012. Production of a Novel Quercetin Glycoside through Metabolic Engineering of Escherichia coli. Appl Environ Microbiol 78:4256-4262.

64. Filippini, M., Kaech, A., Ziegler, U., and Bagheri, H. C. 2011. Fibrisoma limi gen. nov., sp. nov., a filamentous bacterium isolated from tidal flats. Int J Sys Evol Microbiol 61:1418-1424.

65. Lail, K., Sikorski, J., Saunders, E., Lapidus, A., Glavina Del Rio, T., Copeland, A., Tice, H., Cheng, J. F., Lucas,

S., Nolan, M., Bruce, D., Goodwin, L., Pitluck, S., Ivanova, N., Mavromatis, K., Ovchinnikova, G., Pati, A., Chen, A., Palaniappan, K., Land, M., Hauser, L., Chang, Y. J., Jeffries, C. D., Chain, P., Brettin, T., Detter, J. C., Schutze, A., Rohde, M., Tindall, B. J., Goker, M., Bristow, J., Eisen, J. A., Markowitz, V., Hugenholtz, P., Kyrpides, N. C., Klenk, H. P., and Chen, F. 2010. Complete genome sequence of Spirosoma linguale type strain (1). Stand Genom Sci 2:176-185.

66. Lang, E., Lapidus, A., Chertkov, O., Brettin, T., Detter, J. C., Han, C., Copeland, A., Glavina Del Rio, T., Nolan, M., Chen, F., Lucas, S., Tice, H., Cheng, J. F., Land, M., Hauser, L., Chang, Y. J., Jeffries, C. D., Kopitz, M., Bruce, D., Goodwin, L., Pitluck, S., Ovchinnikova, G., Pati, A., Ivanova, N., Mavrommatis, K., Chen, A., Palaniappan, K., Chain, P., Bristow, J., Eisen, J. A., Markowitz, V., Hugenholtz, P., Goker, M., Rohde, M., Kyrpides, N. C., and Klenk, H. P. 2009. Complete genome sequence of Dyadobacter fermentans type strain (NS 114). Stand Genom Sci 1:133-140.

67. Chelius, M. K., and Triplett, E. W. 2000. Dyadobacter fermentans gen. nov., sp. nov., a novel gram-negative bacterium isolated from surface-sterilized Zea mays stems. IntJ Sys Evol Microbiol 50:751-758.

68. Finster, K. W., Herbert, R. A., and Lomstein, B. A. 2009. Spirosoma spitsbergense sp. nov. and Spirosoma luteum sp. nov., isolated from a high Arctic permafrost soil, and emended description of the genus Spirosoma. Int J Sys Evol Microbiol 59:839-844.

69. Filippini, M., Svercel, M., Laczko, E., Kaech, A., Ziegler, U., and Bagheri, H. C. 2011. Fibrella aestuarina gen. nov., sp. nov., a filamentous bacterium of the family Cytophagaceae isolated from a tidal flat, and emended description of the genus Rudanella Weon et al. 2008. Int J Sys Evol Microbiol 61:184-189.

70. Ha, S., Gross, B., and Walker, S. 2001. E. Coli MurG: a paradigm for a superfamily of glycosyltransferases. Curr Drug Targets Infect Disord 1:201-213.

71. Samuel, G., and Reeves, P. 2003. Biosynthesis of O-antigens: genes and pathways involved in nucleotide sugar precursor synthesis and O-antigen assembly. Carbohydr Res 338:2503-2519.

72. Giraud, M. F., and Naismith, J. H. 2000. The rhamnose pathway. Curr Opin Struct Biol 10:687-696.

73. Studier, F. W., Daegelen, P., Lenski, R. E., Maslov, S., and Kim, J. F. 2009. Understanding the Differences between Genome Sequences of Escherichia coli B Strains REL606 and BL21 (DE3) and Comparison of the E. coli B and K-12 Genomes. J Mol Biol 394:653-680.

74. Larbig, K. D., Christmann, A., Johann, A., Klockgether, J., Hartsch, T., Merkl, R., Wiehlmann, L., Fritz, H.-J., and Tümmler, B. 2002. Gene Islands Integrated into tRNAGly Genes Confer Genome Diversity on a Pseudomonas aeruginosa Clone. J Bacteriol 184:6665-6680.

## Claims

**1.** Enzyme catalyzing the glycosylation of polyphenols, wherein the enzyme

a) comprises the amino acid sequence according to SEQ ID NO: 7, or
b) is encoded by a nucleic acid comprising the nucleotide sequence of SEQ ID NO: 1, or
c) is at least 75% homologous to one of the enzymes defined in a) or b) above, or

d) is encoded by a nucleic acid hybridizing under stringent conditions with a nucleic acid complementary to a sequence comprising the nucleotide sequence of SEQ ID NO: 1.

2. Enzyme according to claim 1, wherein the enzyme is at least 80% or 85%, most preferred at least 90%, 95%, 96%, 97%, 98%, 99%, 99.2% or 99.5% homologous to the enzyme defined in a) or b) above.

3. Fragments of an enzyme being at least 97% homologous to the enzyme having the amino acid sequence according to SEQ ID NO. 7, wherein the fragment comprises at least 60, preferably at least 65, 70, 75, 80, 85, 90, 100, 110 or at least 120 consecutive amino acids of said enzyme, and wherein the fragment catalyzes the glycosylation of a polyphenol.

4. Nucleic acid encoding an enzyme according to one of claims 1 to 2, or a fragment according to claim 3.

5. Nuleic acid according to claim 4, wherein the nucleic acid

a) comprises the nucleotide sequence according to SEQ ID NO: 1, or a fragment thereof, the fragment encoding a fragment of an enzyme catalyzing the glycosylation of a polyphenol, or
b) is homologous to one of the nuleic acids defined in a) above, or
c) hybridizes under stringent conditions with a nucleic acid complementary to the nuleic acid defined in a) above.

6. Use of an enzyme according to one of claims 1 to 2, or a fragment thereof according to claim 3, for the glycosylation of polyphenols, preferably phenolic acid derivatives, flavonoids, benzoic acid derivatives, stilbenoids, chalconoids, chromones, and coumarin derivatives.

7. Method for preparing a glycoside of a polyphenol, comprising the step of reacting the polyphenol and a glycosyl donor with an enzyme according to one of claims 1 to 2 or a fragment thereof according to claim 3, under suitable conditions for an enzymatic reaction to occur transferring the glycosyl donor to a hydroxyl group or other functional group of the polyphenol.

8. The method of claim 7, wherein the polyphenol is a phenolic acid derivative, flavonoid, benzoic acid derivative, stilbenoid, chalconoid, chromone, or coumarin derivative.

**Patentansprüche**

1. Enzym, welches die Glykosylierung von Polyphenolen katalysiert, wobei das Enzym

a) die Aminosäuresequenz gemäß SEQ ID NO: 7 umfasst, oder
b) von einer Nukleinsäure kodiert wird, die die Nukleotidsequenz der SEQ ID NO: 1 umfasst, oder
c) mindestens 75% homolog zu einem der oben in a) oder b) definierten Enyzme ist, oder
d) von einer Nukleinsäure kodiert wird, die unter stringenten Bedingunen mit einer Nukleinsäure hybridisiert, die zu einer die Nukleotidsequenz der SEQ ID NO: 1 umfassenden Sequenz komplementär ist.

2. Enzym nach Anspruch 1, wobei das Enzym zu mindestens 80% oder 85%, am meisten bevorzugt zu mindestens 90%, 95%, 96%, 97%, 98%, 99%, 99,2% oder 99,5% homolog zu dem oben in a) oder b) definierten Enzym ist.

3. Fragmente eines Enzyms, das zu mindestens 97% homolog ist zu dem die Aminosäure gemäß SEQ ID NO. 7 aufweisenden Enyzm, wobei das Fragment mindestens 60, vorzugsweise mindestens 65, 70, 75, 80, 85, 90, 100, 110 oder mindestens 120 aufeinanderfolgende Aminosäuren des Enzyms umfasst, und wobei das Fragment die Glykosylierung von Polyphenol katalysiert.

4. Nukleinsäure, die ein Enzym nach einem der Ansprüche 1 bis 2, oder ein Fragment nach Anspruch 3 kodiert.

5. Nukleinsäure nach Anspruch 4, wobei die Nukleinsäure

a) die Nukleotidsequenz gemäß SEQ ID NO: 1, oder ein Fragment davon, umfasst, wobei das Fragment ein Fragment eines Enyzms kodiert, das die Glykosylierung von Poylphenol katalysiert, oder
b) homolog zu einer der oben in a) definierten Nukleinsäuren ist, oder

c) unter stringenten Bedingungen mit einer Nukleinsäure hybridisiert, die zu der oben in a) definierten Nukleinsäure komplementär ist.

6. Verwendung eines Enzyms nach einem der Ansprüche 1 bis 2, oder eines Fragments davon nach Anspruch 3, zur Glykosylierung von Polyphenolen, vorzugsweise Phenolsäurederivaten, Flavonoiden, Benzoesäurederivaten, Stilbenoiden, Chalconoiden, Chromonen und Coumarin-Derivaten.

7. Verfahren zur Herstellung eines Glykosids eines Polyphenols, umfassend den Schritt des Inreaktionbringens des Polyphenols und eines Glykosyldonors mit einem Emyzm nach einem der Ansprüche 1 bis 2 oder einem Fragment davon nach Anspruch 3, unter geeigneten Bedingungen, damit eine enzymatische Reaktion stattfindet, bei der der Glykosyldonor auf eine Hydroxylgruppe oder eine andere funktionelle Gruppe des Poylphenols übertragen wird.

8. Verfahren nach Anspruch 7, wobei das Poylphenol ein Phenolsäurederivat, Flavonoid, Benzoesäurederivat, Stilbenoid, Chalconoid, Chromon oder Coumarin-Derivat ist.

## Revendications

1. Enzyme catalysant la glycosylation de polyphénols, dans laquelle l'enzyme

a) comprend la séquence d'acides aminés selon SEQ ID NO: 7, ou
b) est codée par un acide nucléique comprenant la séquence nucléotidique de SEQ ID NO: 1, ou
c) est au moins à 75 % homologue à une des enzymes définies dans a) ou b) ci-dessus, ou
d) est codée par un acide nucléique s'hybridant dans des conditions stringentes avec un acide nucléique complémentaire à une séquence comprenant la séquence nucléotidique de SEQ ID NO: 1.

2. Enzyme selon la revendication 1, l'enzyme étant au moins à 80 ou 85 %, de préférence entre toutes au moins à 90, 95, 96, 97, 98, 99, 99,2 ou 99,5 % homologue à l'enzyme définie dans a) ou b) ci-dessus.

3. Fragments d'une enzyme étant au moins à 97 % homologue à l'enzyme ayant la séquence d'acides aminés selon SEQ ID NO: 7, dans lesquels le fragment comprend au moins 60, de préférence au moins 65, 70, 75, 80, 85, 90, 100, 110 ou au moins 120 acides aminés consécutifs de la dite enzyme, et dans lesquels le fragment catalyse la glycosylation d'un polyphénol.

4. Acide nucléique codant une enzyme selon une des revendications 1 à 2, ou fragment selon la revendication 3.

5. Acide nucléique selon la revendication 4, dans lequel l'acide nucléique

a) comprend la séquence nucléotidique selon SEQ ID NO: 1, ou un de ses fragments, le fragment codant un fragment d'une enzyme catalysant la glycosylation d'un polyphénol, ou
b) est homologue à un des acides nucléiques définis dans a) ci-dessus, ou
c) s'hybride dans des conditions stringentes avec un acide nucléique complémentaire à l'acide nucléique défini dans a) ci-dessus.

6. Utilisation d'une enzyme selon l'une des revendications 1 à 2, ou d'un de ses fragments selon la revendication 3, pour la glycosylation de polyphénols, de préférence des dérivés d'acide phénolique, de flavonoïdes, de dérivés d'acide benzoïque, de stilbénoïdes, de chalconoïdes, de chromones et de dérivés de coumarine.

7. Procédé de préparation d'un glycoside d'un polyphénol, comprenant l'étape de mise à réagir du polyphénol et d'un donneur glycosyle avec une enzyme selon l'une des revendications 1 à 2 ou un de ses fragments selon la revendication 3, dans des conditions appropriées pour qu'une réaction enzymatique se produise, transférant le donneur glycosyle à un groupe hydroxyle ou à un autre groupe fonctionnel du polyphénol.

8. Procédé selon la revendication 7, dans lequel le polyphénol est un dérivé d'acide phénolique, un flavonoïde, un dérivé d'acide benzoïque, un stilbénoïde, un chalconoïde, une chromone ou un dérivé de coumarine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2128265 A1
- EP 1985704 A1
- EP 1867729 A1
- WO 2009015268 A1
- EP 13169812 A


### Non-patent literature cited in the description


- **HENIKOFF, S. ; HENIKOFF, J.** Amino acid substitution matrices from protein blocks. *Proc Natl. Acad. Sci. USA,* 1992, vol. 89, 10915-10919
- **GREEN M.R. ; SAMBROOK, J.** Molecular Cloning: A Laboratory Manual. Cold Spring Harbor Laboratory Press, 2012
- Gold Book. **A. D. MCNAUGHT ; A. WILKINSON.** IUPAC. Compendium of Chemical Terminology. Blackwell Scientific Publications, 1997
- **A. D. MCNAUGHT ; A. WILKINSON.** IUPAC. Compendium of Chemical Terminology. Blackwell Scientific Publications, 1997
- **ALTSCHUL, S.F. ; GISH, W. ; MILLER, W. ; MYERS, E.W. ; LIPMAN, D.J.** Basic local alignment search tool. *J Mol Biol,* 1990, vol. 215, 403-410
- **HENIKOFF, S. ; HENIKOFF, J.** Amino acid substitution matrices from protein blocks. *Proc Natl Acad Sci USA,* 1992, vol. 89, 10915-10919
- **VERVERIDIS, F. ; TRANTAS, E. ; DOUGLAS, C. ; VOLLMER, G. ; KRETZSCHMAR, G. ; PANOPOULOS, N.** Biotechnology of flavonoids and other phenylpropanoid-derived natural products. Part II: Reconstruction of multienzyme pathways in plants and microbes. *Biotechnol J,* 2007, vol. 2, 1235-1249
- **SCHÜTZ, K. ; MUKS, E. ; CARLE, R. ; SCHIEBER, A.** Quantitative determination of phenolic compounds in artichoke-based dietary supplements and pharmaceuticals by high-performance liquid chromatography. *J Agric Food Chem,* 2006, vol. 54, 8812-8817
- **LEONARD, E. ; YAN, Y. ; FOWLER, Z. L. ; LI, Z. ; LIM, C. G. ; LIM, K. H. ; KOFFAS, M. A.** Strain improvement of recombinant Escherichia coli for efficient production of plant flavonoids. *Mol Pharm,* 2008, vol. 5, 257-265
- **WANG, C. ; MEEK, D. J. ; PANCHAL, P. ; BORUVKA, N. ; ARCHIBALD, F. S ; DRISCOLL, B. T. ; CHARLES, T. C.** Isolation of poly-3-hydroxybutyrate metabolism genes from complex microbial communities by phenotypic complementation of bacterial mutants. *Appl Environ Microbiol,* 2006, vol. 72, 384-391
- **MANACH, C. ; SCALBERT, A. ; MORAND, C. ; REMESY, C. ; JIMENEZ, L.** Polyphenols: food sources and bioavailability. *Am J Clin Nutr,* 2004, vol. 79, 727-747
- **DAS, S. ; ROSAZZA, J. P.** Microbial and enzymatic transformations of flavonoids. *J Nat Prod,* 2006, vol. 69, 499-508
- **GRAEFE, E. U. ; WITTIG, J. ; MUELLER, S. ; RIETHLING, A. K. ; UEHLEKE, B. ; DREWELOW, B. ; PFORTE, H. ; JACOBASCH, G. ; DERENDORF, H. ; VEIT, M.** Pharmacokinetics and bioavailability of quercetin glycosides in humans. *J Clin Pharmacol,* 2001, vol. 41, 492-499
- **KREN, V. ; MARTINKOVA, L.** Glycosides in medicine:The role of glycosidic residue in biological activity. *Curr Med Chem,* 2001, vol. 8, 1303-28
- **COUTINHO, P. M. ; DELEURY, E. ; DAVIES, G. J. ; HENRISSAT, B.** An evolving hierarchical family classification for glycosyltransferases. *J Mol Biol,* 2003, vol. 328, 307-317
- **BOWLES, D. ; LIM, E. K. ; POPPENBERGER, B. ; VAISTIJ, F. E.** Glycosyltransferases of lipophilic small molecules. *Annu Rev Plant Biol,* 2006, vol. 57, 567-597
- **MACKENZIE, P. I. ; OWENS, I. S. ; BURCHELL, B. ; BOCK, K. W. ; BAIROCH, A. ; BELANGER, A. ; FOURNEL-GIGLEUX, S. ; GREEN, M. ; HUM, D. W. ; IYANAGI, T.** The UDP glycosyltransferase gene superfamily: recommended nomenclature update based on evolutionary divergence. *Pharmacogenetics,* 1997, vol. 7, 255-269
- **LAIRSON, L. L. ; HENRISSAT, B. ; DAVIES, G. J. ; WITHERS, S. G.** Glycosyltransferases: structures, functions, and mechanisms. *Annu Rev Biochem,* 2008, vol. 77, 521-555

- **OSMANI, S. A. ; BAK, S. R. ; MOLLER, B. L.** Substrate specificity of plant UDP-dependent glycosyltransferases predicted from crystal structures and homology modeling. *Phytochemistry,* 2009, vol. 70, 325-347
- **BRETON, C. ; SNAJDROVA, L. ; JEANNEAU, C. ; KOCA, J. ; IMBERTY, A.** Structures and mechanisms of glycosyltransferases. *Glycobiology,* 2006, vol. 16, 29R-37
- **YANG, M. ; PROCTOR, M. R. ; BOLAM, D. N. ; ERREY, J. C. ; FIELD, R. A. ; GILBERT, H. J. ; DAVIS, B. G.** Probing the breadth of macrolide glycosyltransferases: in vitro remodeling of a polyketide antibiotic creates active bacterial uptake and enhances potency. *J Am Chem Soc,* 2005, vol. 127, 9336-9337
- **JEON, Y. ; KIM, B. ; KIM, J. ; CHEONG, Y. ; AHN, J.-H.** Enzymatic Glycosylation of Phenolic Compounds Using BsGT-3. *Journal of the Korean Society for Appl Biol Chem,* 2009, vol. 52, 98-101
- **RAO, K. V. ; WEISNER, N. T.** Microbial Transformation of Quercetin by Bacillus cereus. *Appl Environ Microbiol,* 1981, vol. 42, 450-452
- **KIM, H. J. ; KIM, B. G. ; KIM, J. A. ; PARK, Y. ; LEE, Y. J. ; LIM, Y. ; AHN, J. H.** Glycosylation of flavonoids with E. coli expressing glycosyltransferase from Xanthomonas campestris. *J Microbiol Biotechnol,* 2007, vol. 17, 539-542
- **ZHOU, J. ; BRUNS, M. A ; TIEDJE, J. M.** DNA recovery from soils of diverse composition. *Appl Environ Microbiol,* 1996, vol. 62, 316-322
- **ILMBERGER, N. ; MESKE, D. ; JUERGENSEN, J. ; SCHULTE, M. ; BARTHEN, P. ; RABAUSCH, U. ; ANGELOV, A. ; MIENTUS, M ; LIEBL, W. ; SCHMITZ, R. A.** Metagenomic cellulases highly tolerant towards the presence of ionic liquids--linking thermostability and halotolerance. *Appl Microbiol Biotechnol,* 2012, vol. 95, 135-146
- **WAGNER, H. ; BLADT, S. ; ZGAINSKI, E. M.** Drogenanalyse, Dünnschichtchromatographische Analyse von Arzneidrogen. Springer Publisher, 1983
- **NEU, R.** Chelate von Diarylborsäuren mit aliphatischen Oxyalkylaminen als Reagenzien fur den Nachweis von Oxyphenyl-benzo-γ-pyronen. *Naturwissenschaften,* 1957, vol. 44, 181-182
- **TOURASSE, N. J. ; HELGASON, E. ; ØKSTAD, O. A. ; HEGNA, I. K. ; KOLSTØ, A. B.** The Bacillus cereus group: novel aspects of population structure and genome dynamics. *Appl Microbiol,* 2006, vol. 101, 579-593
- **TAMURA K, P. D. ; PETERSON N ; STECHER G ; NEI M ; KUMAR S.** MEGA5: Molecular Evolutionary Genetics Analysis using Maximum Likelihood, Evolutionary Distance, and Maximum Parsimony Methods. *Mol Biol Evol,* 2011, vol. 28, 2731-2739
- **LI, L. ; MODOLO, L. V. ; ESCAMILLA-TREVINO, L. L. ; ACHNINE, L. ; DIXON, R. A. ; WANG, X.** Crystal structure of Medicago truncatula UGT85H2--insights into the structural basis of a multifunctional (iso)flavonoid glycosyltransferase. *J Mol Biol,* 2007, vol. 370, 951-963
- **JONES, P. ; MESSNER, B. ; NAKAJIMA, J.-I. ; SCHÄFFNER, A. R. ; SAITO, K.** UGT73C6 and UGT78D1, Glycosyltransferases Involved in Flavonol Glycoside Biosynthesis in Arabidopsis thaliana. *J Biol Chem,* 2003, vol. 278, 43910-43918
- **KO, J. H. ; GYU KIM, B. ; JOONG-HOON, A.** Glycosylation of flavonoids with a glycosyltransferase from Bacillus cereus. *FEMS Microbiol Lett,* 2006, vol. 258, 263-268
- **JUNG, N. R. ; JOE, E. J. ; KIM, B. G. ; AHN, B. C. ; PARK, J. C. ; CHONG, Y. ; AHN, J. H.** Change of Bacillus cereus flavonoid O-triglucosyltransferase into flavonoid O-monoglucosyltransferase by error-prone polymerase chain reaction. *J Microbiol Biotechnol,* 2010, vol. 20, 1393-1396
- **AHN, B. C. ; KIM, B. G. ; JEON, Y. M. ; LEE, E. J. ; LIM, Y. ; AHN, J. H.** Formation of Flavone Di-O-Glucosides Using a Glycosyltransferase from Bacillus cereus. *J Microbiol Biotechnol,* 2009, vol. 19, 387-390
- **CHOI, S. H. ; RYU, M. ; YOON, Y. J. ; KIM, D. M. ; LEE, E. Y.** Glycosylation of various flavonoids by recombinant oleandomycin glycosyltransferase from Streptomyces antibioticus in batch and repeated batch modes. *Biotechnol Lett,* 2012, vol. 34, 499-505
- **WILLIAMS, G. J. ; ZHANG, C. ; THORSON, J. S.** Expanding the promiscuity of a natural-product glycosyltransferase by directed evolution. *Nat Chem Biol,* 2007, vol. 3, 657-662
- **HU, Y. ; WALKER, S.** Remarkable structural similarities between diverse glycosyltransferases. *Chem Biol,* 2002, vol. 9, 1287-1296
- **HUGHES, J. ; HUGHES, M. A.** Multiple secondary plant product UDP-glucose glucosyltransferase genes expressed in cassava (Manihot esculenta Crantz) cotyledons. *Mitochondrial DNA,* 1994, vol. 5, 41-49
- **PAQUETTE, S. ; MOLLER, B. L. ; BAK, S.** On the origin of family 1 plant glycosyltransferases. *Phytochemistry,* 2003, vol. 62, 399-413
- **BOLAM, D. N. ; ROBERTS, S. ; PROCTOR, M. R. ; TURKENBURG, J. P. ; DODSON, E. J. ; MARTINEZ-FLEITES, C. ; YANG, M. ; DAVIS, B. G. ; DAVIES, G. J. ; GILBERT, H. J.** The crystal structure of two macrolide glycosyltransferases provides a blueprint for host cell antibiotic immunity. *Proc Natl Acad Sci U S A,* 2007, vol. 104, 5336-5341

- **OFFEN, W. ; MARTINEZ-FLEITES, C. ; YANG, M. ; KIAT-LIM, E. ; DAVIS, B. G. ; TARLING, C. A. ; FORD, C. M. ; BOWLES, D. J. ; DAVIES, G. J.** Structure of a flavonoid glucosyltransferase reveals the basis for plant natural product modification. *Embo J,* 2006, vol. 25, 1396-1405
- **MULICHAK, A. M. ; LU, W. ; LOSEY, H. C. ; WALSH, C. T. ; GARAVITO, R. M.** Crystal structure of vancosaminyltransferase GtfD from the vancomycin biosynthetic pathway: interactions with acceptor and nucleotide ligands. *Biochemistry,* 2004, vol. 43, 5170-5180
- **LIM, E.-K. ; ASHFORD, D. A. ; BOWLES, D. J.** The Synthesis of Small-Molecule Rhamnosides through the Rational Design of a Whole-Cell Biocatalysis System. *ChemBioChem,* 2006, vol. 7, 1181-1185
- **MABRY, T. J. ; MARKHAM, K. R. ; THOMAS, M. B.** The systematic identification of flavonoids. New York. Springer-Verlag, 1970
- **GANTT, R. W. ; GOFF, R. D. ; WILLIAMS, G. J. ; THORSON, J. S.** Probing the aglycon promiscuity of an engineered glycosyltransferase. *Angew Chem Int Ed Engl,* 2008, vol. 47, 8889-8892
- **VOGT, T. ; JONES, P.** Glycosyltransferases in plant natural product synthesis: characterization of a supergene family. *Trends Plant Sci,* 2000, vol. 5, 380-386
- **WILLIAMS, G. J. ; GANTT, R. W. ; THORSON, J. S.** The impact of enzyme engineering upon natural product glycodiversification. *Curr Opin Chem Biol,* 2008, vol. 12, 556-564
- **YOON, J. A. ; KIM, B. G. ; LEE, W. J. ; LIM, Y. ; CHONG, Y. ; AHN, J. H.** Production of a Novel Quercetin Glycoside through Metabolic Engineering of Escherichia coli. *Appl Environ Microbiol,* 2012, vol. 78, 4256-4262
- **FILIPPINI, M. ; KAECH, A. ; ZIEGLER, U. ; BAGHERI, H. C.** Fibrisoma limi gen. nov., sp. nov., a filamentous bacterium isolated from tidal flats. *Int J Sys Evol Microbiol,* 2011, vol. 61, 1418-1424
- **LAIL, K. ; SIKORSKI, J. ; SAUNDERS, E. ; LAPIDUS, A. ; GLAVINA DEL RIO, T. ; COPELAND, A. ; TICE, H. ; CHENG, J. F. ; LUCAS, S. ; NOLAN, M.** Complete genome sequence of Spirosoma linguale type strain (1). *Stand Genom Sci,* 2010, vol. 2, 176-185
- **LANG, E. ; LAPIDUS, A. ; CHERTKOV, O. ; BRETTIN, T. ; DETTER, J. C. ; HAN, C. ; COPELAND, A. ; GLAVINA DEL RIO, T. ; NOLAN, M. ; CHEN, F.** Complete genome sequence of Dyadobacter fermentans type strain (NS 114). *Stand Genom Sci,* 2009, vol. 1, 133-140
- **CHELIUS, M. K. ; TRIPLETT, E. W.** Dyadobacter fermentans gen. nov., sp. nov., a novel gram-negative bacterium isolated from surface-sterilized Zea mays stems. *IntJ Sys Evol Microbiol,* 2000, vol. 50, 751-758
- **FINSTER, K. W. ; HERBERT, R. A. ; LOMSTEIN, B. A.** Spirosoma spitsbergense sp. nov. and Spirosoma luteum sp. nov., isolated from a high Arctic permafrost soil, and emended description of the genus Spirosoma. *Int J Sys Evol Microbiol,* 2009, vol. 59, 839-844
- **FILIPPINI, M. ; SVERCEL, M. ; LACZKO, E. ; KAECH, A. ; ZIEGLER, U. ; BAGHERI, H. C. et al.** Fibrella aestuarina gen. nov., sp. nov., a filamentous bacterium of the family Cytophagaceae isolated from a tidal flat, and emended description of the genus. *Int J Sys Evol Microbiol,* 2008, vol. 61, 184-189
- **HA, S. ; GROSS, B. ; WALKER, S.** E. Coli MurG: a paradigm for a superfamily of glycosyltransferases. *Curr Drug Targets Infect Disord,* 2001, vol. 1, 201-213
- **SAMUEL, G. ; REEVES, P.** Biosynthesis of O-antigens: genes and pathways involved in nucleotide sugar precursor synthesis and O-antigen assembly. *Carbohydr Res,* 2003, vol. 338, 2503-2519
- **GIRAUD, M. F. ; NAISMITH, J. H.** The rhamnose pathway. *Curr Opin Struct Biol,* 2000, vol. 10, 687-696
- **STUDIER, F. W. ; DAEGELEN, P. ; LENSKI, R. E. ; MASLOV, S. ; KIM, J. F.** Understanding the Differences between Genome Sequences of Escherichia coli B Strains REL606 and BL21 (DE3) and Comparison of the E. coli B and K-12 Genomes. *J Mol Biol,* 2009, vol. 394, 653-680
- **LARBIG, K. D. ; CHRISTMANN, A. ; JOHANN, A. ; KLOCKGETHER, J. ; HARTSCH, T. ; MERKL, R. ; WIEHLMANN, L. ; FRITZ, H.-J. ; TÜMMLER, B.** Gene Islands Integrated into tRNAGly Genes Confer Genome Diversity on a Pseudomonas aeruginosa Clone. *J Bacteriol,* 2002, vol. 184, 6665-6680